Europäisches Patentamt

European Patent Office

Office européen des brevets

(19)

(11) Numéro de publication: **0 325 527**
**A2**

## (12) DEMANDE DE BREVET EUROPEEN

(21) Numéro de dépôt: 89400134.6

(22) Date de dépôt: 17.01.89

(51) Int. Cl.⁴: **G 06 F 15/20**

(30) Priorité: 19.01.88 FR 8800545

(43) Date de publication de la demande:
26.07.89 Bulletin 89/30

(84) Etats contractants désignés:
AT BE CH DE ES GB GR IT LI LU NL SE

(71) Demandeur: INSTITUT NATIONAL DE LA SANTE ET DE
LA RECHERCHE MEDICALE (INSERM)
101, rue de Tolbiac
F-75654 Paris Cédex 13 (FR)

(72) Inventeur: Pernier, Jacques
25, Rue Louise Michel
F-69800 Saint Priest (FR)

Perrin, François
92, Chemin de Montray
F-69110 Sainte Foy (FR)

(74) Mandataire: Bugnon-Hays, Claudine
PATCO S.A. 10, rue Vivienne
F-75002 Paris (FR)

(54) Procédé et dispositif de cartographie cérébrale par méthode d'interpolation.

(57) La présente invention concerne un procédé d'interpolation d'une valeur électrique U en un point quelconque d'une sphère à partir d'un nombre fini n de mesures effectuées sur des sites $P_i$ déterminés et connus.

Elle concerne également un dispositif comportant un circuit de traitement des signaux recueillis en des sites d'acquisition connus, un calculateur central muni de moyens de mémoire et des moyens de visualisation.

Applications : topographie des potentiels évoqués, neurologie.

EP 0 325 527 A2

**Description**

### Procédé et dispositif de cartographie cérébrale par méthode d'interpolation

La présente invention concerne un procédé d'interpolation d'une grandeur quelconque en un point quelconque d'une sphère, ainsi qu'un dispositif de détermination de ladite valeur électrique et en un dispositif de visualisation de la topographie d'un champ de potentiel.

Elle concerne, en particulier, les examens d'exploration fonctionnelle en neurologie, consistant à extraire de l'électroencéphalogramme la réponse électrique du cerveau provoquée par l'application au sujet d'une stimulation physiologique (stimulation sonore, visuelle, somesthésique). Cette réponse est recueillie à l'aide de plusieurs électrodes réparties sur le scalp.

Selon l'état de la technique, on utilise un nombre élevé d'électrodes réparties régulièrement sur la totalité de la surface sphérique. L'estimation des mesures entre deux sites d'acquisition se fait par des méthodes d'interpolation ne permettant pas d'obtenir une précision suffisante.

La présente invention concerne un procédé d'interpolation permettant d'obtenir avec un nombre peu élevé d'électrodes réparties en des sites d'acquisition quelconques la valeur d'un champ d'une grandeur électromagnétique, notamment d'un champ d'un potentiel ou d'un courant électrique, ou d'une grandeur magnétique, par exemple la composante radiale du champ magnétique.

La présente invention concerne plus particulièrement un procédé d'interpolation d'une valeur électrique U en un point P quelconque d'une sphère prise comme modèle de la partie supérieure de la tête, à partir d'un nombre fini n de mesures $Z_i$ $U(P_i)$ effectuées en les n sites $P_i$ déterminés et connus, consistant :

- à stocker dans une mémoire d'ordinateur les coordonnées polaires $(\theta_i, \varphi_i)$ des sites $P_i$, ainsi que les valeurs associées $Z_i$ du signal électrique en un temps t donné,
- à calculer pour les n sites de mesure $P_i$ les valeurs :

$$x_i = \cos \varphi_i$$
$$y_i = \sin \varphi_i$$
$$et \quad z_i = \sqrt{1 - x_i^2 - y_i^2} \quad si \ \theta_i \leqslant \frac{\pi}{2}$$
$$- \sqrt{1 - x_i^2 - y_i^2} \quad si \ \theta_i > \frac{\pi}{2}$$

- à calculer pour tous les couples de mesures $(P_i, P_j)$ la distance :

$$d_{ij} = \sqrt{(x_i - x_j)^2 + (y_i - y_j)^2 + (z_i - z_j)^2}$$

et la valeur :

$$\cos \gamma_{ij} = 1 - \frac{d^2_{ij}}{2}$$

- à calculer la valeur de la fonction $k_m$ $(P_i, P_j)$ par extraction de la valeur de la fonction $k_m$ dans un tableau pré-calculé,
- à construire la matrice :

$$\begin{bmatrix} K & | & E \\ ----- & | & ----- \\ E^t & | & O \end{bmatrix}$$

où E est le vecteur $(1, 1, ... 1)^t$ et K la matrice des valeurs $k_m (P_i, P_j)$,
- à déterminer la valeur des coéfficients :
$P = (p_1, ... p_n)^t$ et le coéfficient q par résolution de l'équation

$$
\begin{bmatrix} K & | & E \\ \text{---} & | & \text{---} \\ E^t & | & O \end{bmatrix}
\quad X \quad
\begin{bmatrix} P \\ \text{---} \\ q \end{bmatrix}
\quad = \quad
\begin{bmatrix} Z \\ \text{---} \\ O \end{bmatrix}
$$

Z étant le tableau $(Z_1, ... Z_n)^t$,
- à calculer pour tous les points P' de coordonnées (X, Y) du plan de projection de la sphère les valeurs :

$$\theta = \sqrt{X^2 + Y^2}$$

$$x = X \ \frac{\sin \theta}{\theta}$$

$$y = Y \ \frac{\sin \theta}{\theta}$$

$$z = \sqrt{1 - x^2 - y^2} \quad \text{si} \ \theta \leqslant \frac{\pi}{2}$$

$$\quad - \sqrt{1 - x^2 - y^2} \quad \text{si} \ \theta > \frac{\pi}{2}$$

si on adopte la méthode de projection radiale,
- à déterminer le coéfficient :

$$\cos \gamma (P, P_i) = 1 - \frac{d^2}{2}$$

$$\text{où} \ d = \sqrt{(x - x_i)^2 + (y - y_i)^2 + (z - z_i)^2}$$

- à déterminer $k_m (\cos \gamma (P, P_i))$ par extraction de la valeur de la fonction $k_m$ dans un tableau pré-calculé,
- à calculer la valeur électrique U :

$$U = \sum_{i = 1}^{n} P_i \cdot k_m (P, P_i) + q$$

$$\text{où} \quad k_m (P, P_i) = \frac{1}{4\pi} \sum_{\gamma = 1}^{\infty} \frac{2\gamma + 1}{\gamma^m (\gamma + 1)^m} \left[ P_\gamma \cos \gamma (P, P_i) \right]$$

et $P\nu = \nu$ième polynôme de Legendre.
   Selon un mode de mise en oeuvre préféré, on stocke dans la mémoire d'un ordinateur 2001 valeurs de la fonction $k_m$ pour $\cos \gamma$ variant de -1 à +1 et on détermine une valeur $k_m(\cos \gamma)$ par extraction de la valeur stockée à l'adresse partie entière de (1000 cos $\gamma$ + 1001).

Selon une variante préférée, la résolution de l'équation matricielle :

$$
\begin{bmatrix} K & \vdots & E \\ ----- & \vdots & ----- \\ E^t & \vdots & O \end{bmatrix}
\quad X \quad
\begin{bmatrix} P \\ --- \\ q \end{bmatrix}
\quad = \quad
\begin{bmatrix} Z \\ --- \\ O \end{bmatrix}
$$

est obtenue par l'utilisation d'un sous-programme de factorisation de matrice symétrique avec pivots sur la diagonale.

La présente invention concerne, en particulier, un procédé de visualisation de la topographie d'un champ de potentiel ou magnétique consistant :
- à recueillir le signal en un nombre fini de sites,
- à interpoler les valeurs du signal entre lesdits sites selon le procédé décrit ci-dessus,
- à générer pour chaque pixel de visualisation un signal représentatif du signal interpolé correspondant.

Le procédé selon l'invention autorise un traitement rapide et permet de représenter l'évolution au cours du temps des signaux recueillis sur une sphère et interpolés entre les sites d'acquisition.

Il permet, notamment, de visualiser l'évolution dans le temps du champ de potentiel représentant la réponse électrique du cerveau provoquée par une stimulation physiologique.

La présente invention peut, en particulier, être appliquée pour la reconstitution de la topographie du champ de potentiel évoqué consistant :
- à disposer sur le scalp d'un sujet un nombre fini d'électrodes,
- à interpoler la valeur du potentiel pour tous les autres points de la topographie,
- à visualiser la valeur desdits potentials sur un écran selon une projection plane du scalp.

Selon une variante, la présente invention permet la reconstruction de la topographie de la composante radiale du vecteur densité de courant au voisinage de la surface du scalp, à partir des valeurs $Z_i$ des potentiels électriques mesurés en un nombre fini d'électrodes.

Le procédé consiste :
- à stocker dans une mémoire d'ordinateur les coordonnées polaires $(\theta_i, \varphi_i)$ des sites $P_i$, ainsi que les valeurs associées $Z_i$ du signal électrique en un temps t donné,
- à calculer pour les n sites de mesure $P_i$ les valeurs :

$x_i = \cos \varphi_i$

$y_i = \sin \varphi_i$

$$
\text{et } z_i = \sqrt{1 - x_i^2 - y_i^2} \quad \text{si } \theta \leqslant \frac{\pi}{2}
$$

$$
- \sqrt{1 - x_i^2 - y_i^2} \quad \text{si } \theta > \frac{\pi}{2}
$$

- à calculer pour tous les couples de mesure $(P_i, P_j)$ la distance :

$$
d_{ij} = \sqrt{(x_i - x_j)^2 + (y_i - y_j)^2 + (z_i - z_j)^2}
$$

et la valeur :

$$
\cos \gamma_{ij} = 1 - \frac{d^2_{ij}}{2}
$$

- à calculer la valeur de la fonction $k_m (P_i, P_j)$ par extraction de la valeur de la fonction $k_m$ dans un tableau pré-calculé,
- à construire la matrice :

4

$$\begin{bmatrix} K & \vdots & E \\ \cdots & \vdots & \cdots \\ E^t & \vdots & O \end{bmatrix}$$

où E est le vecteur $(1, 1, 1, \ldots 1)^t$ et K la matrice des valeurs $k_m$ $(P_i, P_j)$,
- à déterminer la valeur des coéfficients :
$P = (p_1, \ldots p_n)^t$ et le coéfficient q par résolution de l'équation :

$$\begin{bmatrix} K & \vdots & E \\ \cdots & \vdots & \cdots \\ E^t & \vdots & O \end{bmatrix} \times \begin{bmatrix} P \\ \cdots \\ q \end{bmatrix} = \begin{bmatrix} Z \\ \cdots \\ O \end{bmatrix}$$

Z étant le tableau $(Z_1, \ldots Z_n)^t$,
- à calculer pour tous les points P' de coordonnées (X, Y) du plan de projection de la sphère les valeurs :

$$\theta = \sqrt{X^2 + Y^2}$$

$$x = X \frac{\sin \theta}{\theta}$$

$$y = Y \frac{\sin \theta}{\theta}$$

$$z = \sqrt{1 - x^2 - y^2} \quad \text{si } \theta \leqslant \frac{\pi}{2}$$

$$\quad - \sqrt{1 - x^2 - y^2} \quad \text{si } \theta > \frac{\pi}{2}$$

- à calculer la valeur de la composante radiale du vecteur :

$$J_m(p) = - \sum_{i=1}^{n} p_i \, h_m \, (P, P_i)$$

avec cette fois-ci :

$$h_m(P, P_i) = \frac{1}{4\pi} \sum_{\nu=1}^{\infty} \frac{2\nu + 1}{\nu^{m-1}(\nu+1)^{m-1}} \, P_\nu \left[ \cos \gamma (P, P_i) \right]$$

$h_m$ étant extrait d'un tableau pré-calculé.

La présente invention concerne également un dispositif de détermination de la valeur d'un signal électrique en un site quelconque d'une sphère, comportant des circuits de traitement du signal électrique recueilli en un nombre fini de sites d'acquisition, un calculateur muni de moyens de mémorisation commandés par un programme mettant en oeuvre le procédé décrit ci-dessus, ainsi que des moyens de visualisation de la valeur du signal électrique correspondant au site considéré.

Le dispositif selon l'invention est, en particulier, destiné à la visualisation du champ de potentiels représentant un paramètre électroencéphalographique comportant des circuits de traitement du signal recueilli par un nombre fini d'électrodes, un calculateur commandé par un programme pour le calcul des valeurs d'interpolation selon le procédé de l'invention, des moyens de mémorisation et des moyens de visualisation.

D'autres avantages et modes de réalisation ressortiront mieux de la description qui va suivre, s'appuyant sur les dessins où :

- la figure 1 représente une vue en perspective d'un scalp et d'un site de mesure $P_i$ et d'un point P où l'on cherche à estimer la valeur de la mesure,
- la figure 2 représente l'organigramme du programme de mise en oeuvre du procédé,
- la figure 3 représente le tableau pré-calculé des valeurs $k_m$,

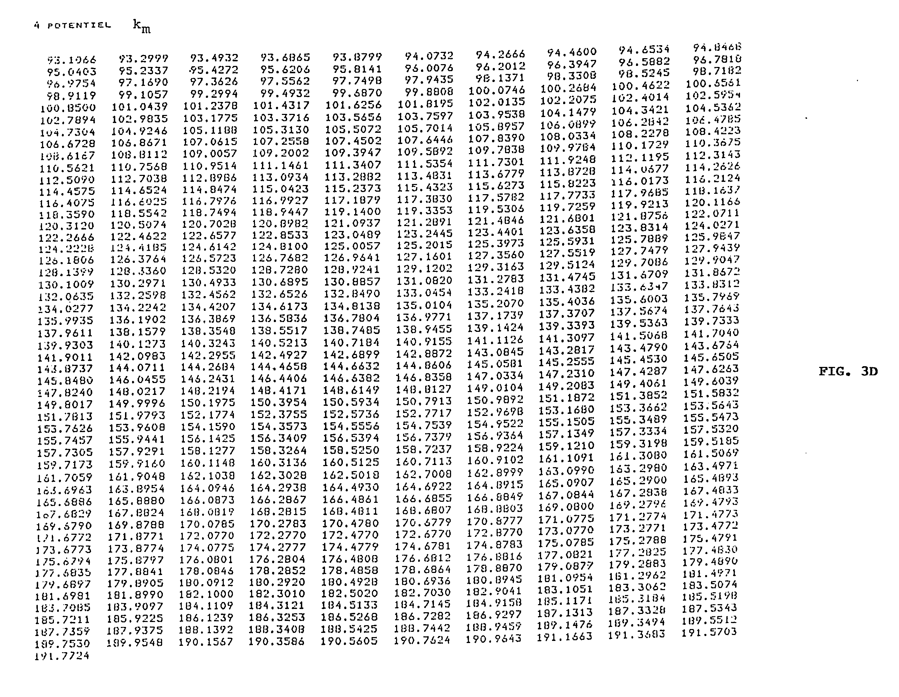

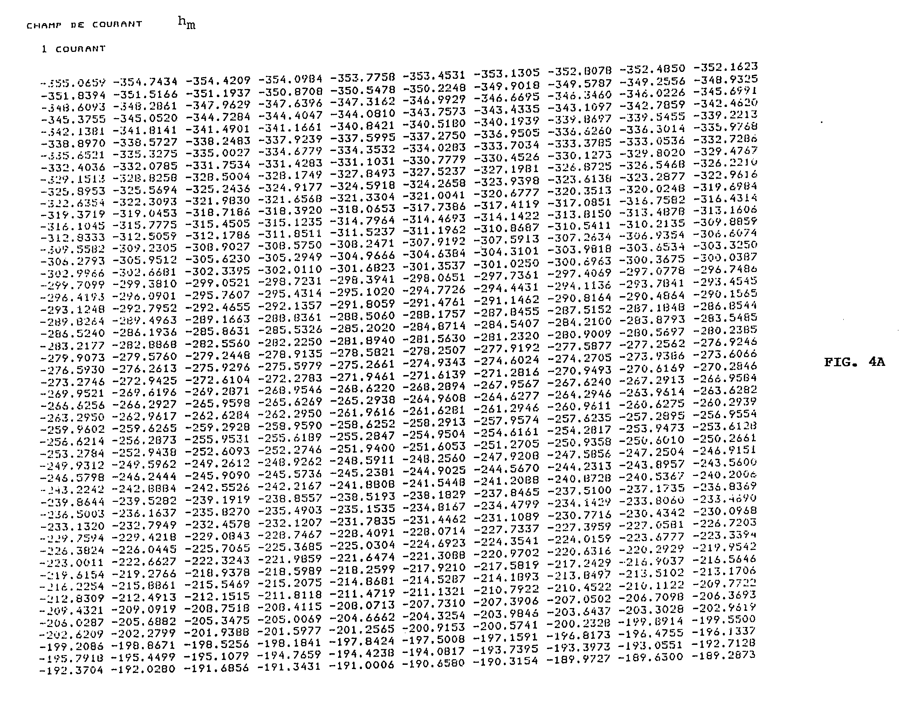

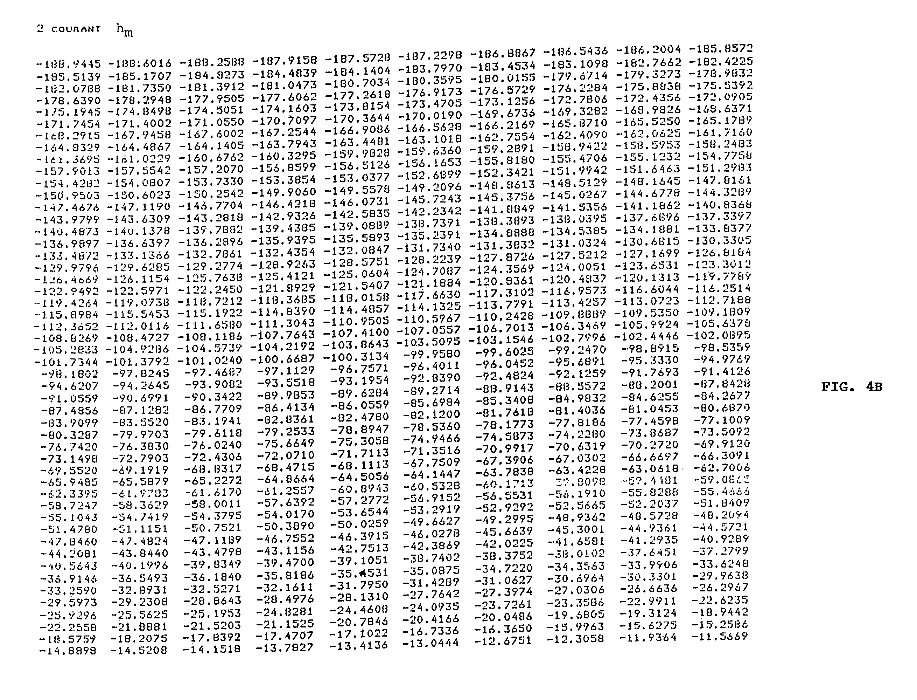

- la figure 4 représente le tableau pré-calculé des valeurs $h_m$,

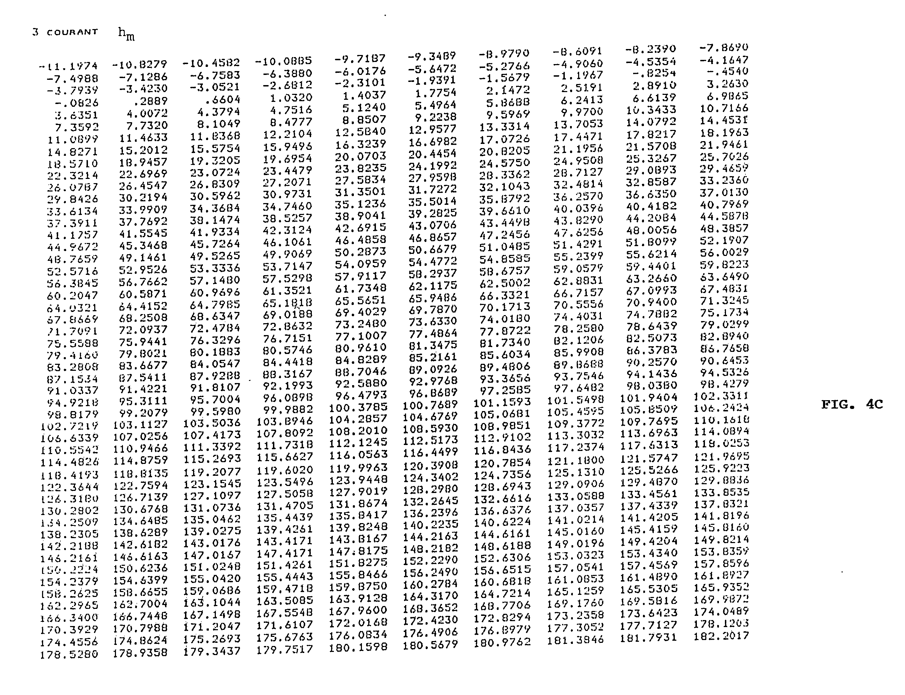

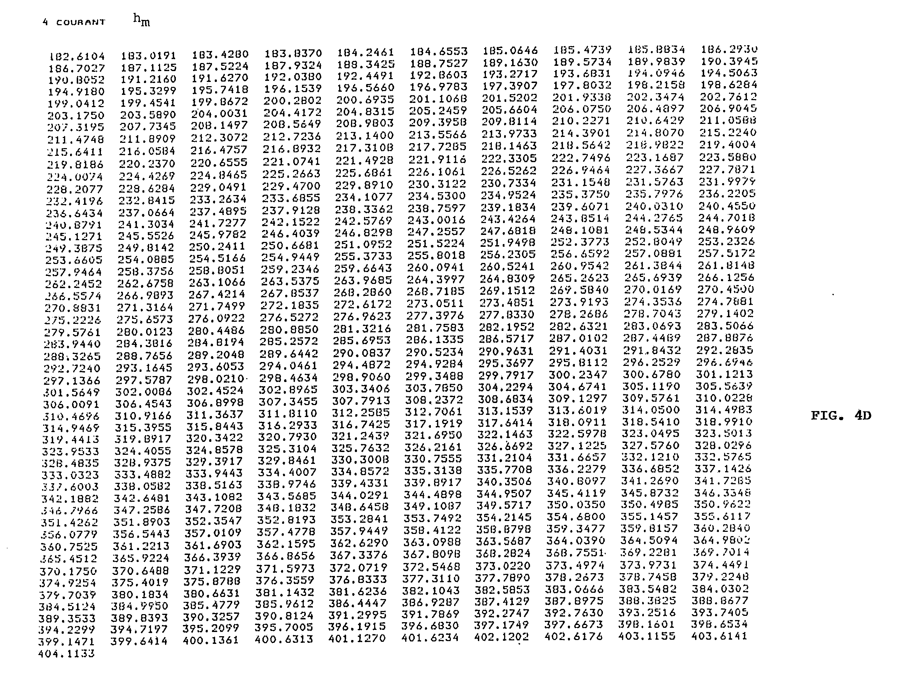

- la figure 5 représente le schéma de principe d'un dispositif pour la mise en oeuvre de l'invention.

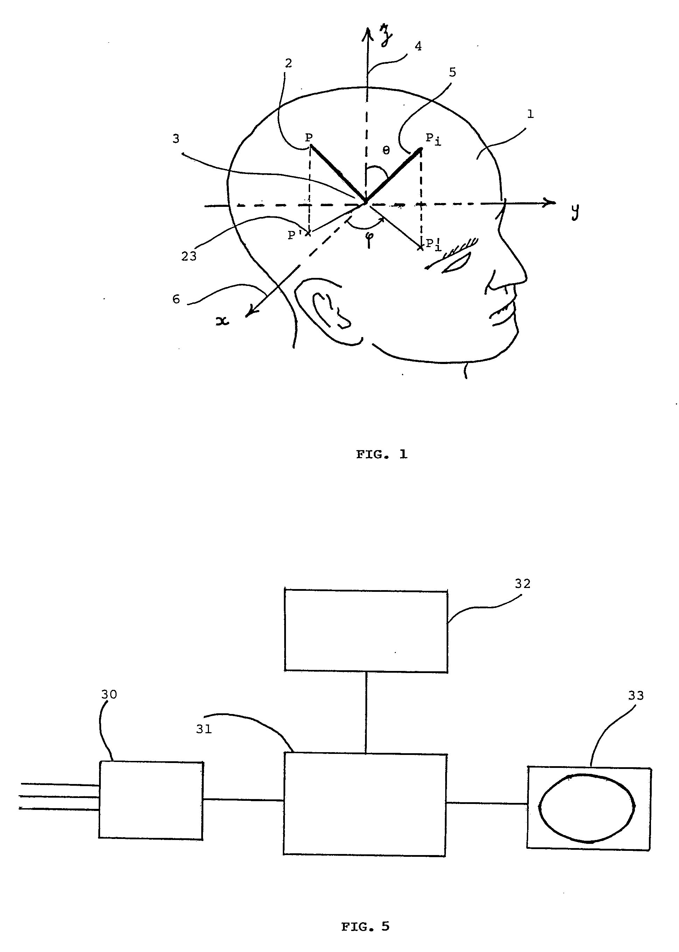

On dispose d'un certain nombre de valeurs $Z_i$ mesurées sur des sites $P_i$ répartis sur le scalp d'un sujet, qu'on assimile à une sphère, tel que représenté en figure 1.

Les mesures sont recueillies par des électrodes au nombre de 16 à 32 par exemple, réparties sur les points $P_i$ du scalp (1).

Le problème à résoudre est d'estimer la valeur U du signal électrique en un point P quelconque (2) du scalp (1) en fonction des valeurs $U(P_i)$ mesurées et éventuellement de projeter ces informations sur un plan pour visualiser la répartition topographique des valeurs ainsi calculées.

Les points $P_i$ du scalp (1) sont repérés par leurs coordonnées polaires ($\theta$, $\varphi$), $\theta$ représentant l'angle que fait le vecteur joignant le centre de la sphère (3) avec le point $P_i$ avec un axe appelé OZ (4) et $\varphi$ représentant l'angle que fait la projection du vecteur OP (5) dans le plan XOY perpendiculaire à OZ, avec un axe appelé OX (6). L'orientation de OZ (4) peut être choisie quelconque. Sur la figure 1, elle est verticale et l'axe passe par le sommet de la tête.

Le procédé selon l'invention consiste en une interpolation à partir des positions de mesure $P_i$ ($\theta_i$, $\varphi_i$) et des valeurs associées $Z_i$, à estimer la valeur U associée à n'importe quel point P sur la surface de la sphère.

Le plan de projection XOY est découpé en une matrice de carrés élémentaires destinés à représenter un pixel sur un écran d'ordinateur. Le centre de chaque carré élémentaire représentera un point P' du plan (XOY).

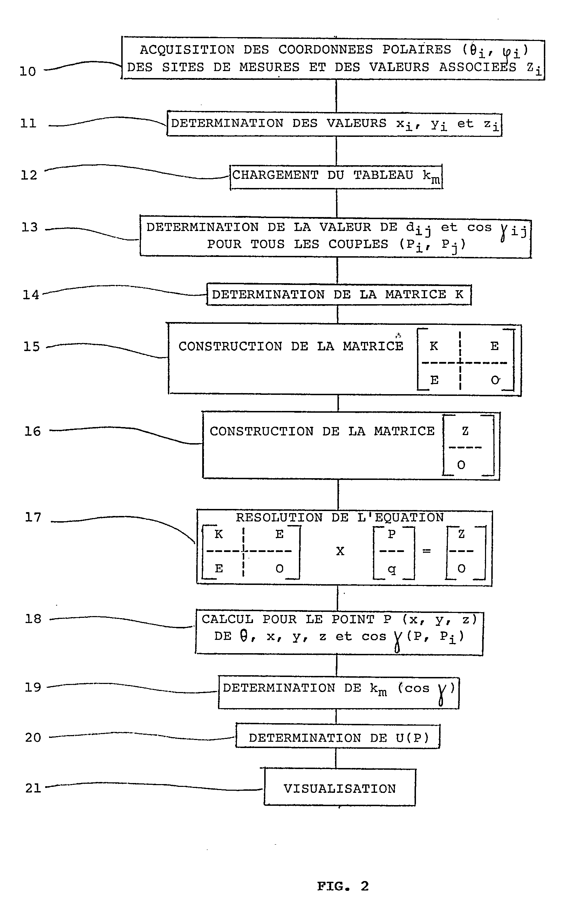

Le procédé consiste tout d'abord à acquérir les coordonnées de tous les sites de mesure, ainsi que les valeurs mesurées $Z_i$ associées. Cette opération correspond à l'étape 10 de l'algorithme représenté en figure 2.

L'étape 11 consiste à déterminer les valeurs $x_i$, $y_i$ et $Z_i$ des sites de mesure où :

$$x_i = \cos \varphi_i$$
$$y_i = \sin \varphi_i$$
$$\text{et } Z_i = \sqrt{1 - x_i^2 - y_i^2} \quad \text{si } \theta \leqslant \frac{\pi}{2}$$
$$-\sqrt{1 - x_i^2 - y_i^2} \quad \text{si } \theta > \frac{\pi}{2}$$

L'étape 12 consiste à charger le tableau des valeurs $k_m$ en mémoire sur l'ordinateur. Cette étape peut intervenir à un moment quelconque de l'algorithme.

Les valeurs $k_m$ peuvent être calculées par la formule :

6

$$k_m (P, P_i) = \frac{1}{4\pi} \sum_{\nu = 1}^{\infty} \frac{2\nu + 1}{\nu^m (\nu + 1)^m} \times P_\nu \left[ \cos \gamma (P, P_i) \right]$$

où P est le vième polynôme de Legendre et $(P, P_i)$ l'angle que font les vecteurs OP et $OP_i$.

Pour diminuer la quantité de calculs et, donc, accélérer la procédure de synthèse d'images, il est avantageux de calculer à l'avance les valeurs $k_m$ en fonction de $\cos \gamma$ et de les stocker dans une mémoire.

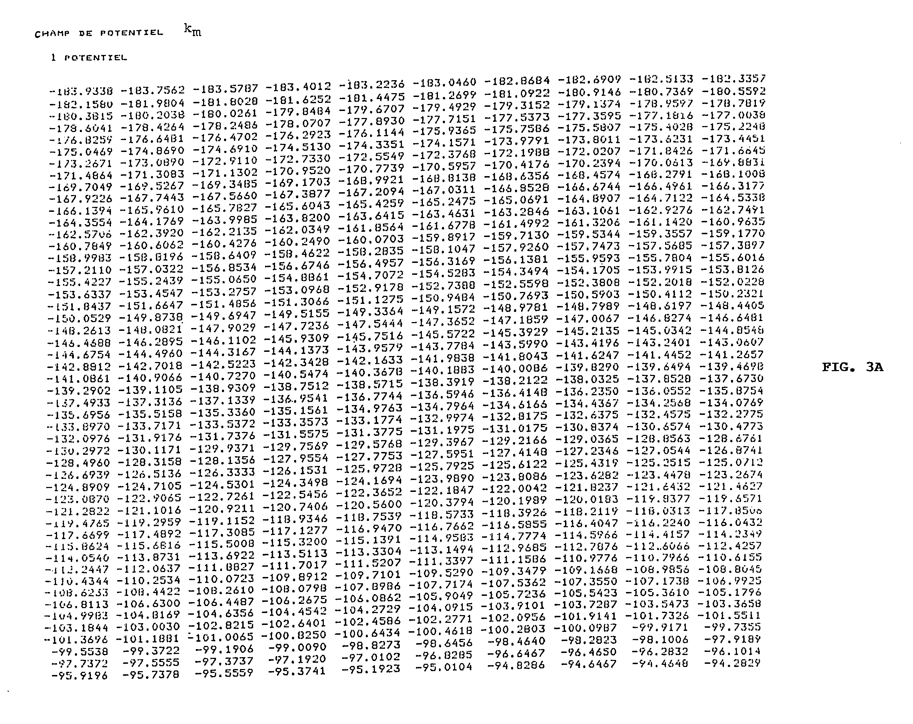

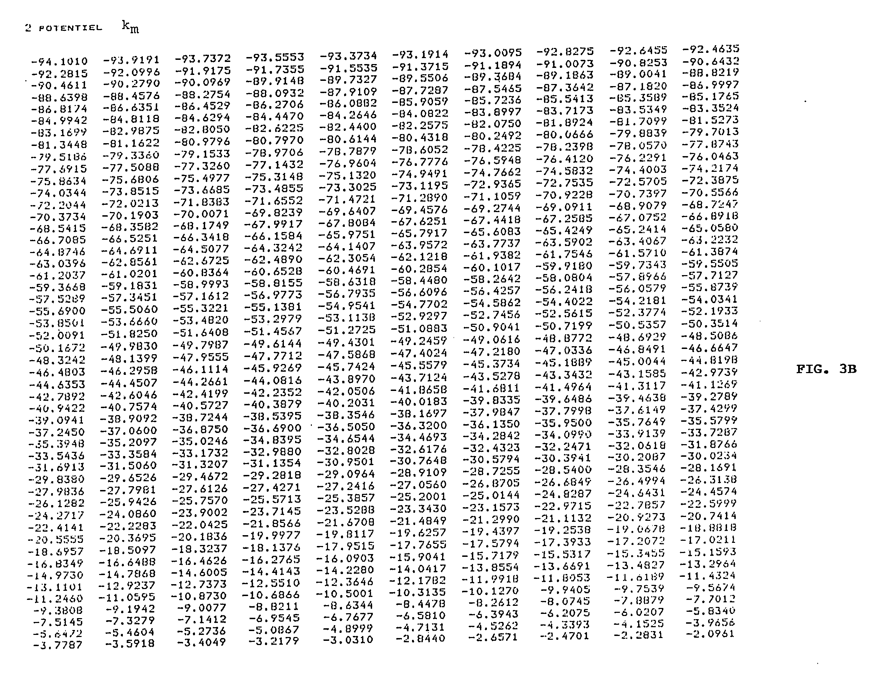

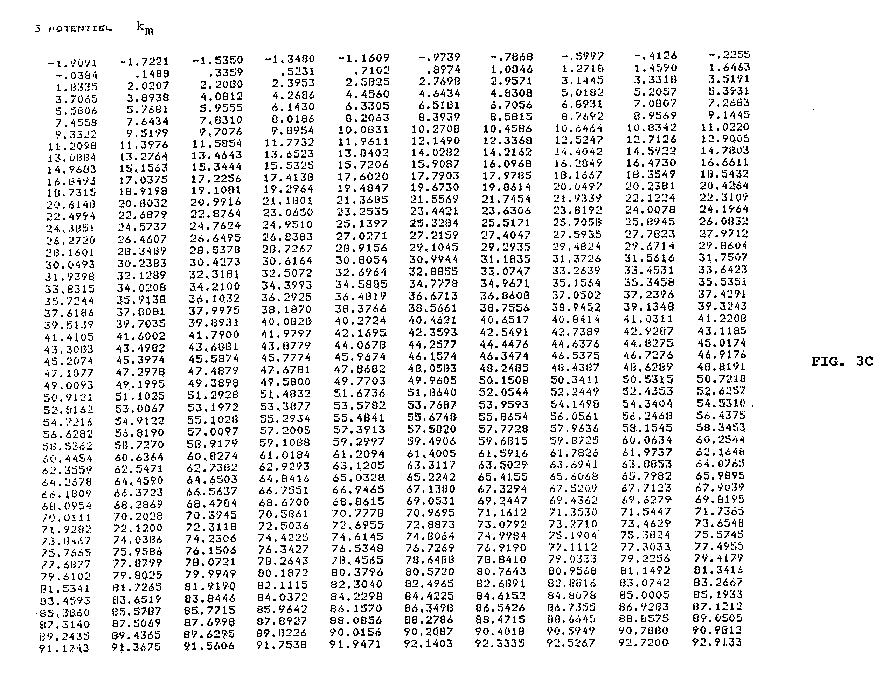

La figure 3 représente, à titre d'exemple, un tableau comportant 2001 valeurs de $k_m$ pour $\cos \gamma$ variant de -1 à +1.

L'étape 13 consiste à déterminer la valeur $d_{ij}$ pour chacun des sites de mesure $(P_i, P_j)$ :

$$d_{ij} = \sqrt{(x_i - x_j)^2 + (y_i - y_j)^2 + (z_i - z_j)^2}$$

ainsi que la valeur $\cos \gamma_{ij} = 1 - \dfrac{d^2_{ij}}{2}$ .

L'étape 14 consiste à déterminer la matrice K des valeurs $k_{ij}$.

La valeur $k_{ij}$ sera déterminée par extraction de la valeur $k_m$ dont l'adresse correspond à la partie entière de $(1000 \cos \gamma_{ij} + 1001)$.

On pourra éventuellement affiner la valeur de $k_{ij}$ en faisant une extrapolation linéaire entre deux valeurs du tableau lorsque $1000 \cos \gamma_{ij}$ n'est pas entier.

L'étape 15 consiste à construire une matrice :

$$\begin{bmatrix} K & \vdots & E \\ \cdots & \vdots & \cdots \\ E^t & \vdots & O \end{bmatrix}$$

où K est la matrice des valeurs $k_{ij}$, E le tableau $(1, 1, \ldots 1)^t$.

L'étape 16 consiste à construire la matrice :

$$\begin{bmatrix} Z \\ \cdots \\ O \end{bmatrix}$$

où Z est le vecteur des valeurs $Z_i$ mesurées.

L'étape 17 consiste à résoudre l'équation matricielle :

$$\begin{bmatrix} K & \vdots & E \\ ----- & \vdots & ----- \\ E^t & \vdots & O \end{bmatrix} \times \begin{bmatrix} P \\ --- \\ q \end{bmatrix} = \begin{bmatrix} z \\ ---- \\ 0 \end{bmatrix}.$$

La résolution de cette équation matricielle peut être obtenue en utilisant un sous-programme de factorisation de matrice symétrique avec pivots sur la diagonale. On pourra, notamment, recourir aux routines SSPFA et SSPSL du logiciel LINPACK.

L'étape 18 consiste à calculer pour un point P (2) de coordonnées cartésiennes (x, y, z) les valeurs $\theta$, x, y, z et $\cos \gamma$ (P, $P_i$) où (X, Y) sont les coordonnées de la projection P' (23) du point P (2) sur le plan de projection (XOY) et correspondant au centre d'un pixel de visualisation sur un écran. Si, par exemple, P' est obtenu par une projection radiale :

$$\theta = \sqrt{X^2 + Y^2}$$

$$x = X \; \frac{\sin \theta}{\theta}$$

$$y = Y \; \frac{\sin \theta}{\theta}$$

$$z = \sqrt{1 - x^2 - y^2} \quad \text{si } \dot{\theta} \leqslant \frac{\pi}{2}$$

$$- \sqrt{1 - x^2 - y^2} \quad \text{si } \theta > \frac{\pi}{2}$$

$$\cos \gamma \, (P, \; P_i) = 1 - \frac{d^2}{2}$$

$$\text{où} \quad d^2 = \sqrt{(x - x_i)^2 + (y - y_i)^2 + (z - z_i)^2}$$

Dans toute la description, on considère que les coordonnées sont déterminées dans un système normé, la distance entre un point quelconque du scalp (1) et le centre O (3) étant normée à 1.

L'étape 18 consiste à déterminer la valeur de $k_m$ (P, $P_i$) par extraction de la valeur de $k_m$ ($\cos \gamma$ (P, $P_i$)) selon le procédé déjà décrit ou par calcul.

L'étape 19 consiste à déterminer la fonction d'interpolation $U_m(P)$ où :

$$U_m(p) = \sum_{i=1}^{n} P_i \times k_m \, (P, \; P_i) + q$$

$P_i$ et q étant les solutions de l'équation matricielle résolue à l'étape 17.

Ce procédé d'interpolation d'un signal électrique sera mis en oeuvre pour représenter la distribution topographique d'une grandeur recueillie sur des sites répartis sur le scalp du sujet.

La présente invention concerne également un dispositif pour la mise en oeuvre du procédé précédemment décrit.

Le dispositif comporte, en particulier, des circuits de traitement (30) des signaux électriques acquis sur les sites de mesure. Ces circuits peuvent, notamment, comporter des amplificateurs, des digitaliseurs et des systèmes d'élimination des bruits de fond et des perturbations diverses.

Ces signaux traités sont transmis à un calculateur central (31) comportant des circuits mémoires (32).

Ces circuits mémoires (32) sont, notamment, destinés à stocker le tableau des valeurs pré-calculées $k_m$, ainsi que les coordonnées des sites de mesure $P_i$.

Le calculateur (31) est commandé par un programme destiné à la mise en oeuvre du procédé selon l'invention.

Un système de visualisation (33) - un écran ou une table traçante -permet de représenter la topographie du signal ainsi que l'évolution dans le temps.

La présente invention n'est pas limitée aux modes de réalisation et exemples d'application et de mise en oeuvre décrits mais s'étend, au contraire, à toutes les variantes.

## Revendications

1) Dispositif de détermination de la valeur d'une valeur électrique $U_m(P)$ en un site quelconque P d'une sphère, à partir d'un nombre fini de mesures $Z_i$ effectuées en des sites $P_i$ déterminés et connus, caractérisé en ce qu'il comporte des moyens de mesure de la valeur $Z_i$ du signal électrique disposés sur les dites $P_i$ de coordonnées $(\theta_i, \varphi_i)$ et un calculateur comportant une mémoire de stockage des coordonnées $(\theta_i, \varphi_i)$ et des valeurs du signal électrique $Z_i$ associées calculant pour les n sites de $P_i$ de mesure les valeurs :

$$x_i = \cos \varphi_i$$
$$y_i = \sin \varphi_i$$
$$et \quad z_i = \sqrt{1 - x_i^2 - y_i^2} \quad si \ \theta \leqslant \frac{\pi}{2}$$
$$- \sqrt{1 - x_i^2 - y_i^2} \quad si \ \theta > \frac{\pi}{2}$$

et pour tous les couples de sites de mesure $(P_i, P_j)$ :

$$d_{ij} = \sqrt{(x - x_i)^2 + (y - y_i)^2 + (z - z_i)^2}$$
$$et \quad \cos \gamma_{ij} = 1 - \frac{d^2_{ij}}{2} \quad ,$$

ainsi que la valeur de la fonction $k_m (\cos \gamma_{ij})$ par extraction de la valeur de la fonction $k_m$ dans un tableau pré-calculé, ledit calculateur construisant la matrice :

$$\left[ \begin{array}{c|c} K & E \\ \hline E^t & O \end{array} \right]$$

E étant le tableau $(1, 1, ... 1)^t$ et déterminant la valeur des coefficients $P = (p_1, ... p_n)^t$ et q par résolution de la matrice :

-9

$$
\begin{bmatrix} K & \vdots & E \\ \cdots & \cdots & \cdots \\ E^t & \vdots & O \end{bmatrix} \times \begin{bmatrix} p \\ \cdots \\ q \end{bmatrix} = \begin{bmatrix} z \\ \cdots \\ O \end{bmatrix}
$$

$Z$ étant le tableau $(Z_1, \dots Z_n)^t$ et calculant pour tous les points $P'$ de coordonnées $(X, Y)$ du plan de projection les valeurs, dans le cas d'un mode de projection de type radial :

$$
\theta = \sqrt{X^2 + Y^2}
$$

$$
x = X \frac{\sin \theta}{\theta}
$$

$$
y = Y \frac{\sin \theta}{\theta}
$$

$$
z = \sqrt{1 - x^2 - y^2} \quad \text{si } \theta \leqslant \frac{\pi}{2}
$$

$$
- \sqrt{1 - x^2 - y^2} \quad \text{si } \theta > \frac{\pi}{2}
$$

ledit calculateur déterminant en outre le coefficient :

$$
\cos \gamma(P, P_i) = 1 - \frac{d^2}{2}
$$

$$
\text{avec } d = \sqrt{(x - x_i)^2 + (y - y_i)^2 + (z - z_i)^2},
$$

et la variable $k_m (\cos \gamma)$ par extraction de la valeur de la fonction $k_m$ dans un tableau pré-calculé et affichant la valeur :

$$
U_m (P) = \sum_{i = 1}^{n} p_i \cdot k_m + q
$$

correspondant à la valeur du signal électrique au point P.

2) Dispositif de visualisation de la topographie du champ de potentiel représentant un paramètre électroencéphalographique caractérisé en ce qu'il comporte des circuits de traitement du signal recueilli par un nombre fini d'électrodes placées sur le scalp en des sites $P_i$, un calculateur commandé par un programme pour le calcul des valeurs interpolées $U_m(P)$ calculant, à partir des coordonnées $(\theta_i, \varphi_i)$ des sites $P_i$ d'implantation des électrodes et des mesures $Z_i$ du signal mesuré en ces sites $P_i$, les variables :

$$x_i = \cos \varphi_i$$

$$y_i = \sin \varphi_i$$

et $z_i = \sqrt{1 - x_i^2 - y_i^2}$ si $\theta \leqslant \dfrac{\pi}{2}$

$-\sqrt{1 - x_i^2 - y_i^2}$ si $\theta > \dfrac{\pi}{2}$

et pour tous les couples de sites de mesure $(P_i, P_j)$ :

$$d_{ij} = \sqrt{(x - x_i)^2 + (y - y_i)^2 + (z - z_i)^2}$$

et $\cos \gamma_{ij} = 1 - \dfrac{d^2_{ij}}{2}$ ,

ainsi que la valeur de la fonction $k_m$ ($\cos \gamma_{ij}$) par extraction de la valeur de la fonction $k_m$ dans un tableau pré-calculé, ledit calculateur construisant la matrice :

$$\begin{bmatrix} K & \vline & E \\ \hline E^t & \vline & 0 \end{bmatrix}$$

E étant le tableau $(1, 1, \ldots 1)^t$ et déterminant la valeur des coefficients $P = (p_1 \ldots p_n)^t$ et q par résolution de la matrice :

$$\begin{bmatrix} K & \vline & E \\ \hline E^t & \vline & 0 \end{bmatrix} \times \begin{bmatrix} p \\ \hline q \end{bmatrix} = \begin{bmatrix} z \\ \hline 0 \end{bmatrix}$$

Z étant le tableau $(Z_1, \ldots Z_n)^t$ et calculant pour tous les points $P'$ de coordonnées $(X, Y)$ du plan de projection les valeurs, dans le cas d'un mode de projection de type radial :

$$\theta = \sqrt{x^2 + y^2}$$

$$x = X \frac{\sin \theta}{\theta}$$

$$y = Y \frac{\sin \theta}{\theta}$$

$$z = \sqrt{1 - x^2 - y^2} \quad \text{si } \theta \leqslant \frac{\pi}{2}$$

$$- \sqrt{1 - x^2 - y^2} \quad \text{si } \theta > \frac{\pi}{2}$$

ledit calculateur déterminant en outre le coefficient :

$$\cos\gamma(P, P_i) = 1 - \frac{d^2}{2}$$

$$\text{avec } d = \sqrt{(x - x_i)^2 + (y - y_i)^2 + (z - z_i)^2},$$

et la variable $k_m$ (cos$\gamma$) par extraction de la valeur de la fonction $k_m$ dans un tableau pré-calculé et affichant la valeur :

$$U_m(p) = \sum_{i=1}^{n} p_i \cdot k_m + q$$

correspondant à la valeur du signal électrique au point P,
ledit dispositif de visualisation comportant en outredes moyens de mémorisation des valeurs $U_m(P)$ et des moyens de visualisation de la topographie des valeurs $U_m(P)$ déterminées par ledit calculateur sur une projection plane du scalp.

3) Dispositif selon l'une quelconque des revendications 1 à 2, caractérisé en ce que ledit calculateur comporte, en outre, une mémoire de stockage des valeurs de la fonction $k_m$(cos$\gamma$)précalculées pour cos$\gamma$ compris entre -1 et +1.

4) Dispositif selon l'une quelconque des revendications 1 à 3, caractérisé en ce que le calculateur détermine la valeur $h_m(P, P_i)$ par extraction de la fonction $h_m$ dans un tableau précalculé et calcule la composante radiale du vecteur densité du courant :

$$J_m(P) = - \sum_{i=1}^{n} p_i \cdot h_m(P, P_i).$$

12

FIG. 1

FIG. 5

ACQUISITION DES COORDONNEES POLAIRES $(\theta_i, \varphi_i)$
DES SITES DE MESURES ET DES VALEURS ASSOCIEES $z_i$

DETERMINATION DES VALEURS $x_i$, $y_i$ et $z_i$

CHARGEMENT DU TABLEAU $k_m$

DETERMINATION DE LA VALEUR DE $d_{ij}$ et cos $\gamma_{ij}$
POUR TOUS LES COUPLES $(P_i, P_j)$

DETERMINATION DE LA MATRICE K

CONSTRUCTION DE LA MATRICE $\begin{bmatrix} K & E \\ \hline E & 0 \end{bmatrix}$

CONSTRUCTION DE LA MATRICE $\begin{bmatrix} Z \\ \hline 0 \end{bmatrix}$

RESOLUTION DE L'EQUATION
$\begin{bmatrix} K & E \\ \hline E & 0 \end{bmatrix} \times \begin{bmatrix} P \\ \hline q \end{bmatrix} = \begin{bmatrix} Z \\ \hline 0 \end{bmatrix}$

CALCUL POUR LE POINT P $(x, y, z)$
DE $\theta$, $x$, $y$, $z$ et cos $\gamma(P, P_i)$

DETERMINATION DE $k_m$ (cos $\gamma$)

DETERMINATION DE U(P)

VISUALISATION

FIG. 2

1 POTENTIEL

```
-183.9338 -183.7562 -183.5787 -183.4012 -183.2236 -183.0460 -182.8684 -182.6909 -182.5133 -182.3357
-182.1580 -181.9804 -181.8028 -181.6252 -181.4475 -181.2699 -181.0922 -180.9146 -180.7369 -180.5592
-180.3815 -180.2038 -180.0261 -179.8484 -179.6707 -179.4929 -179.3152 -179.1374 -178.9597 -178.7819
-178.6041 -178.4264 -178.2486 -178.0707 -177.8930 -177.7151 -177.5373 -177.3595 -177.1816 -177.0038
-176.8259 -176.6481 -176.4702 -176.2923 -176.1144 -175.9365 -175.7586 -175.5807 -175.4028 -175.2248
-175.0469 -174.8690 -174.6910 -174.5130 -174.3351 -174.1571 -173.9791 -173.8011 -173.6231 -173.4451
-173.2671 -173.0890 -172.9110 -172.7330 -172.5549 -172.3768 -172.1988 -172.0207 -171.8426 -171.6645
-171.4864 -171.3083 -171.1302 -170.9520 -170.7739 -170.5957 -170.4176 -170.2394 -170.0613 -169.8831
-169.7049 -169.5267 -169.3485 -169.1703 -168.9921 -168.8138 -168.6356 -168.4574 -168.2791 -168.1008
-167.9226 -167.7443 -167.5660 -167.3877 -167.2094 -167.0311 -166.8528 -166.6744 -166.4961 -166.3177
-166.1394 -165.9610 -165.7827 -165.6043 -165.4259 -165.2475 -165.0691 -164.8907 -164.7122 -164.5338
-164.3554 -164.1769 -163.9985 -163.8200 -163.6415 -163.4631 -163.2846 -163.1061 -162.9276 -162.7491
-162.5706 -162.3920 -162.2135 -162.0349 -161.8564 -161.6778 -161.4992 -161.3206 -161.1420 -160.9635
-160.7849 -160.6062 -160.4276 -160.2490 -160.0703 -159.8917 -159.7130 -159.5344 -159.3557 -159.1770
-158.9983 -158.8196 -158.6409 -158.4622 -158.2835 -158.1047 -157.9260 -157.7473 -157.5685 -157.3897
-157.2110 -157.0322 -156.8534 -156.6746 -156.4957 -156.3169 -156.1381 -155.9593 -155.7804 -155.6016
-155.4227 -155.2439 -155.0650 -154.8861 -154.7072 -154.5283 -154.3494 -154.1705 -153.9915 -153.8126
-153.6337 -153.4547 -153.2757 -153.0968 -152.9178 -152.7388 -152.5598 -152.3808 -152.2018 -152.0228
-151.8437 -151.6647 -151.4856 -151.3066 -151.1275 -150.9484 -150.7693 -150.5903 -150.4112 -150.2321
-150.0529 -149.8738 -149.6947 -149.5155 -149.3364 -149.1572 -148.9781 -148.7989 -148.6197 -148.4405
-148.2613 -148.0821 -147.9029 -147.7236 -147.5444 -147.3652 -147.1859 -147.0067 -146.8274 -146.6481
-146.4688 -146.2895 -146.1102 -145.9309 -145.7516 -145.5722 -145.3929 -145.2135 -145.0342 -144.8548
-144.6754 -144.4960 -144.3167 -144.1373 -143.9579 -143.7784 -143.5990 -143.4196 -143.2401 -143.0607
-142.8912 -142.7018 -142.5223 -142.3428 -142.1633 -141.9838 -141.8043 -141.6247 -141.4452 -141.2657
-141.0861 -140.9066 -140.7270 -140.5474 -140.3678 -140.1883 -140.0086 -139.8290 -139.6494 -139.4698
-139.2902 -139.1105 -138.9309 -138.7512 -138.5715 -138.3919 -138.2122 -138.0325 -137.8528 -137.6730
-137.4933 -137.3136 -137.1339 -136.9541 -136.7744 -136.5946 -136.4148 -136.2350 -136.0552 -135.8754
-135.6956 -135.5158 -135.3360 -135.1561 -134.9763 -134.7964 -134.6166 -134.4367 -134.2568 -134.0769
-133.8970 -133.7171 -133.5372 -133.3573 -133.1774 -132.9974 -132.8175 -132.6375 -132.4575 -132.2775
-132.0976 -131.9176 -131.7376 -131.5575 -131.3775 -131.1975 -131.0175 -130.8374 -130.6574 -130.4773
-130.2972 -130.1171 -129.9371 -129.7569 -129.5768 -129.3967 -129.2166 -129.0365 -128.8563 -128.6761
-128.4960 -128.3158 -128.1356 -127.9554 -127.7753 -127.5951 -127.4148 -127.2346 -127.0544 -126.8741
-126.6939 -126.5136 -126.3333 -126.1531 -125.9728 -125.7925 -125.6122 -125.4319 -125.2515 -125.0712
-124.8909 -124.7105 -124.5301 -124.3498 -124.1694 -123.9890 -123.8086 -123.6282 -123.4478 -123.2674
-123.0870 -122.9065 -122.7261 -122.5456 -122.3652 -122.1847 -122.0042 -121.8237 -121.6432 -121.4627
-121.2822 -121.1016 -120.9211 -120.7406 -120.5600 -120.3794 -120.1989 -120.0183 -119.8377 -119.6571
-119.4765 -119.2959 -119.1152 -118.9346 -118.7539 -118.5733 -118.3926 -118.2119 -118.0313 -117.8506
-117.6699 -117.4892 -117.3085 -117.1277 -116.9470 -116.7662 -116.5855 -116.4047 -116.2240 -116.0432
-115.8624 -115.6816 -115.5008 -115.3200 -115.1391 -114.9583 -114.7774 -114.5966 -114.4157 -114.2349
-114.0540 -113.8731 -113.6922 -113.5113 -113.3304 -113.1494 -112.9685 -112.7876 -112.6066 -112.4257
-112.2447 -112.0637 -111.8827 -111.7017 -111.5207 -111.3397 -111.1586 -110.9776 -110.7966 -110.6155
-110.4344 -110.2534 -110.0723 -109.8912 -109.7101 -109.5290 -109.3479 -109.1668 -108.9856 -108.8045
-108.6233 -108.4422 -108.2610 -108.0798 -107.8986 -107.7174 -107.5362 -107.3550 -107.1738 -106.9925
-106.8113 -106.6300 -106.4487 -106.2675 -106.0862 -105.9049 -105.7236 -105.5423 -105.3610 -105.1796
-104.9983 -104.8169 -104.6356 -104.4542 -104.2729 -104.0915 -103.9101 -103.7287 -103.5473 -103.3658
-103.1844 -103.0030 -102.8215 -102.6401 -102.4586 -102.2771 -102.0956 -101.9141 -101.7326 -101.5511
-101.3696 -101.1881 -101.0065 -100.8250 -100.6434 -100.4618 -100.2803 -100.0987  -99.9171  -99.7355
 -99.5538  -99.3722  -99.1906  -99.0090  -98.8273  -98.6456  -98.4640  -98.2823  -98.1006  -97.9189
 -97.7372  -97.5555  -97.3737  -97.1920  -97.0102  -96.8285  -96.6467  -96.4650  -96.2832  -96.1014
 -95.9196  -95.7378  -95.5559  -95.3741  -95.1923  -95.0104  -94.8286  -94.6467  -94.4648  -94.2829
```

FIG. 3A

EP 0 325 527 A2

2 POTENTIEL $k_m$

| | | | | | | | | | |
|---|---|---|---|---|---|---|---|---|---|
| -94.1010 | -93.9191 | -93.7372 | -93.5553 | -93.3734 | -93.1914 | -93.0095 | -92.8275 | -92.6455 | -92.4635 |
| -92.2815 | -92.0996 | -91.9175 | -91.7355 | -91.5535 | -91.3715 | -91.1894 | -91.0073 | -90.8253 | -90.6432 |
| -90.4611 | -90.2790 | -90.0969 | -89.9148 | -89.7327 | -89.5506 | -89.3684 | -89.1863 | -89.0041 | -88.8219 |
| -88.6398 | -88.4576 | -88.2754 | -88.0932 | -87.9109 | -87.7287 | -87.5465 | -87.3642 | -87.1820 | -86.9997 |
| -86.8174 | -86.6351 | -86.4529 | -86.2706 | -86.0882 | -85.9059 | -85.7236 | -85.5413 | -85.3589 | -85.1765 |
| -84.9942 | -84.8118 | -84.6294 | -84.4470 | -84.2646 | -84.0822 | -83.8997 | -83.7173 | -83.5349 | -83.3524 |
| -83.1699 | -82.9875 | -82.8050 | -82.6225 | -82.4400 | -82.2575 | -82.0750 | -81.8924 | -81.7099 | -81.5273 |
| -81.3448 | -81.1622 | -80.9796 | -80.7970 | -80.6144 | -80.4318 | -80.2492 | -80.0666 | -79.8839 | -79.7013 |
| -79.5186 | -79.3360 | -79.1533 | -78.9706 | -78.7879 | -78.6052 | -78.4225 | -78.2398 | -78.0570 | -77.8743 |
| -77.6915 | -77.5088 | -77.3260 | -77.1432 | -76.9604 | -76.7776 | -76.5948 | -76.4120 | -76.2291 | -76.0463 |
| -75.8634 | -75.6806 | -75.4977 | -75.3148 | -75.1320 | -74.9491 | -74.7662 | -74.5832 | -74.4003 | -74.2174 |
| -74.0344 | -73.8515 | -73.6685 | -73.4855 | -73.3025 | -73.1195 | -72.9365 | -72.7535 | -72.5705 | -72.3875 |
| -72.2044 | -72.0213 | -71.8383 | -71.6552 | -71.4721 | -71.2890 | -71.1059 | -70.9228 | -70.7397 | -70.5566 |
| -70.3734 | -70.1903 | -70.0071 | -69.8239 | -69.6407 | -69.4576 | -69.2744 | -69.0911 | -68.9079 | -68.7247 |
| -68.5415 | -68.3582 | -68.1749 | -67.9917 | -67.8084 | -67.6251 | -67.4418 | -67.2585 | -67.0752 | -66.8918 |
| -66.7085 | -66.5251 | -66.3418 | -66.1584 | -65.9751 | -65.7917 | -65.6083 | -65.4249 | -65.2414 | -65.0580 |
| -64.8746 | -64.6911 | -64.5077 | -64.3242 | -64.1407 | -63.9572 | -63.7737 | -63.5902 | -63.4067 | -63.2232 |
| -63.0396 | -62.8561 | -62.6725 | -62.4890 | -62.3054 | -62.1218 | -61.9382 | -61.7546 | -61.5710 | -61.3874 |
| -61.2037 | -61.0201 | -60.8364 | -60.6528 | -60.4691 | -60.2854 | -60.1017 | -59.9180 | -59.7343 | -59.5505 |
| -59.3668 | -59.1831 | -58.9993 | -58.8155 | -58.6318 | -58.4480 | -58.2642 | -58.0804 | -57.8966 | -57.7127 |
| -57.5289 | -57.3451 | -57.1612 | -56.9773 | -56.7935 | -56.6096 | -56.4257 | -56.2418 | -56.0579 | -55.8739 |
| -55.6900 | -55.5060 | -55.3221 | -55.1381 | -54.9541 | -54.7702 | -54.5862 | -54.4022 | -54.2181 | -54.0341 |
| -53.8501 | -53.6660 | -53.4820 | -53.2979 | -53.1138 | -52.9297 | -52.7456 | -52.5615 | -52.3774 | -52.1933 |
| -52.0091 | -51.8250 | -51.6408 | -51.4567 | -51.2725 | -51.0883 | -50.9041 | -50.7199 | -50.5357 | -50.3514 |
| -50.1672 | -49.9830 | -49.7987 | -49.6144 | -49.4301 | -49.2459 | -49.0616 | -48.8772 | -48.6929 | -48.5086 |
| -48.3242 | -48.1399 | -47.9555 | -47.7712 | -47.5868 | -47.4024 | -47.2180 | -47.0336 | -46.8491 | -46.6647 |
| -46.4803 | -46.2958 | -46.1114 | -45.9269 | -45.7424 | -45.5579 | -45.3734 | -45.1889 | -45.0044 | -44.8198 |
| -44.6353 | -44.4507 | -44.2661 | -44.0816 | -43.8970 | -43.7124 | -43.5278 | -43.3432 | -43.1585 | -42.9739 |
| -42.7892 | -42.6046 | -42.4199 | -42.2352 | -42.0506 | -41.8658 | -41.6811 | -41.4964 | -41.3117 | -41.1269 |
| -40.9422 | -40.7574 | -40.5727 | -40.3879 | -40.2031 | -40.0183 | -39.8335 | -39.6486 | -39.4638 | -39.2789 |
| -39.0941 | -38.9092 | -38.7244 | -38.5395 | -38.3546 | -38.1697 | -37.9847 | -37.7998 | -37.6149 | -37.4299 |
| -37.2450 | -37.0600 | -36.8750 | -36.6900 | -36.5050 | -36.3200 | -36.1350 | -35.9500 | -35.7649 | -35.5799 |
| -35.3948 | -35.2097 | -35.0246 | -34.8395 | -34.6544 | -34.4693 | -34.2842 | -34.0990 | -33.9139 | -33.7287 |
| -33.5436 | -33.3584 | -33.1732 | -32.9880 | -32.8028 | -32.6176 | -32.4323 | -32.2471 | -32.0618 | -31.8766 |
| -31.6913 | -31.5060 | -31.3207 | -31.1354 | -30.9501 | -30.7648 | -30.5794 | -30.3941 | -30.2087 | -30.0234 |
| -29.8380 | -29.6526 | -29.4672 | -29.2818 | -29.0964 | -28.9109 | -28.7255 | -28.5400 | -28.3546 | -28.1691 |
| -27.9836 | -27.7981 | -27.6126 | -27.4271 | -27.2416 | -27.0560 | -26.8705 | -26.6849 | -26.4994 | -26.3138 |
| -26.1282 | -25.9426 | -25.7570 | -25.5713 | -25.3857 | -25.2001 | -25.0144 | -24.8287 | -24.6431 | -24.4574 |
| -24.2717 | -24.0860 | -23.9002 | -23.7145 | -23.5288 | -23.3430 | -23.1573 | -22.9715 | -22.7857 | -22.5999 |
| -22.4141 | -22.2283 | -22.0425 | -21.8566 | -21.6708 | -21.4849 | -21.2990 | -21.1132 | -20.9273 | -20.7414 |
| -20.5555 | -20.3695 | -20.1836 | -19.9977 | -19.8117 | -19.6257 | -19.4397 | -19.2538 | -19.0678 | -18.8818 |
| -18.6957 | -18.5097 | -18.3237 | -18.1376 | -17.9515 | -17.7655 | -17.5794 | -17.3933 | -17.2072 | -17.0211 |
| -16.8349 | -16.6488 | -16.4626 | -16.2765 | -16.0903 | -15.9041 | -15.7179 | -15.5317 | -15.3455 | -15.1593 |
| -14.9730 | -14.7868 | -14.6005 | -14.4143 | -14.2280 | -14.0417 | -13.8554 | -13.6691 | -13.4827 | -13.2964 |
| -13.1101 | -12.9237 | -12.7373 | -12.5510 | -12.3646 | -12.1782 | -11.9918 | -11.8053 | -11.6189 | -11.4324 |
| -11.2460 | -11.0595 | -10.8730 | -10.6866 | -10.5001 | -10.3135 | -10.1270 | -9.9405 | -9.7539 | -9.5674 |
| -9.3808 | -9.1942 | -9.0077 | -8.8211 | -8.6344 | -8.4478 | -8.2612 | -8.0745 | -7.8879 | -7.7012 |
| -7.5145 | -7.3279 | -7.1412 | -6.9545 | -6.7677 | -6.5810 | -6.3943 | -6.2075 | -6.0207 | -5.8340 |
| -5.6472 | -5.4604 | -5.2736 | -5.0867 | -4.8999 | -4.7131 | -4.5262 | -4.3393 | -4.1525 | -3.9656 |
| -3.7787 | -3.5918 | -3.4049 | -3.2179 | -3.0310 | -2.8440 | -2.6571 | -2.4701 | -2.2831 | -2.0961 |

FIG. 3B

EP 0 325 527 A2

3 POTENTIEL $k_m$

| | | | | | | | | | |
|---|---|---|---|---|---|---|---|---|---|
| -1.9091 | -1.7221 | -1.5350 | -1.3480 | -1.1609 | -.9739 | -.7868 | -.5997 | -.4126 | -.2255 |
| -.0384 | .1489 | .3359 | .5231 | .7102 | .8974 | 1.0846 | 1.2718 | 1.4590 | 1.6463 |
| 1.8335 | 2.0207 | 2.2080 | 2.3953 | 2.5825 | 2.7698 | 2.9571 | 3.1445 | 3.3318 | 3.5191 |
| 3.7065 | 3.8938 | 4.0812 | 4.2686 | 4.4560 | 4.6434 | 4.8308 | 5.0182 | 5.2057 | 5.3931 |
| 5.5806 | 5.7681 | 5.9555 | 6.1430 | 6.3305 | 6.5181 | 6.7056 | 6.8931 | 7.0807 | 7.2683 |
| 7.4558 | 7.6434 | 7.8310 | 8.0186 | 8.2063 | 8.3939 | 8.5815 | 8.7692 | 8.9569 | 9.1445 |
| 9.3322 | 9.5199 | 9.7076 | 9.8954 | 10.0831 | 10.2708 | 10.4586 | 10.6464 | 10.8342 | 11.0220 |
| 11.2098 | 11.3976 | 11.5854 | 11.7732 | 11.9611 | 12.1490 | 12.3368 | 12.5247 | 12.7126 | 12.9005 |
| 13.0884 | 13.2764 | 13.4643 | 13.6523 | 13.8402 | 14.0282 | 14.2162 | 14.4042 | 14.5922 | 14.7803 |
| 14.9683 | 15.1563 | 15.3444 | 15.5325 | 15.7206 | 15.9087 | 16.0968 | 16.2849 | 16.4730 | 16.6611 |
| 16.8493 | 17.0375 | 17.2256 | 17.4138 | 17.6020 | 17.7903 | 17.9785 | 18.1667 | 18.3549 | 18.5432 |
| 18.7315 | 18.9198 | 19.1081 | 19.2964 | 19.4847 | 19.6730 | 19.8614 | 20.0497 | 20.2381 | 20.4264 |
| 20.6148 | 20.8032 | 20.9916 | 21.1801 | 21.3685 | 21.5569 | 21.7454 | 21.9339 | 22.1224 | 22.3109 |
| 22.4994 | 22.6879 | 22.8764 | 23.0650 | 23.2535 | 23.4421 | 23.6306 | 23.8192 | 24.0078 | 24.1964 |
| 24.3851 | 24.5737 | 24.7624 | 24.9510 | 25.1397 | 25.3284 | 25.5171 | 25.7058 | 25.8945 | 26.0832 |
| 26.2720 | 26.4607 | 26.6495 | 26.8383 | 27.0271 | 27.2159 | 27.4047 | 27.5935 | 27.7823 | 27.9712 |
| 28.1601 | 28.3489 | 28.5378 | 28.7267 | 28.9156 | 29.1045 | 29.2935 | 29.4824 | 29.6714 | 29.8604 |
| 30.0493 | 30.2383 | 30.4273 | 30.6164 | 30.8054 | 30.9944 | 31.1835 | 31.3726 | 31.5616 | 31.7507 |
| 31.9398 | 32.1289 | 32.3181 | 32.5072 | 32.6964 | 32.8855 | 33.0747 | 33.2639 | 33.4531 | 33.6423 |
| 33.8315 | 34.0208 | 34.2100 | 34.3993 | 34.5885 | 34.7778 | 34.9671 | 35.1564 | 35.3458 | 35.5351 |
| 35.7244 | 35.9138 | 36.1032 | 36.2925 | 36.4819 | 36.6713 | 36.8608 | 37.0502 | 37.2396 | 37.4291 |
| 37.6186 | 37.8081 | 37.9975 | 38.1870 | 38.3766 | 38.5661 | 38.7556 | 38.9452 | 39.1348 | 39.3243 |
| 39.5139 | 39.7035 | 39.8931 | 40.0828 | 40.2724 | 40.4621 | 40.6517 | 40.8414 | 41.0311 | 41.2208 |
| 41.4105 | 41.6002 | 41.7900 | 41.9797 | 42.1695 | 42.3593 | 42.5491 | 42.7389 | 42.9287 | 43.1185 |
| 43.3083 | 43.4982 | 43.6881 | 43.8779 | 44.0678 | 44.2577 | 44.4476 | 44.6376 | 44.8275 | 45.0174 |
| 45.2074 | 45.3974 | 45.5874 | 45.7774 | 45.9674 | 46.1574 | 46.3474 | 46.5375 | 46.7276 | 46.9176 |
| 47.1077 | 47.2978 | 47.4879 | 47.6781 | 47.8682 | 48.0583 | 48.2485 | 48.4387 | 48.6289 | 48.8191 |
| 49.0093 | 49.1995 | 49.3898 | 49.5800 | 49.7703 | 49.9605 | 50.1508 | 50.3411 | 50.5315 | 50.7218 |
| 50.9121 | 51.1025 | 51.2928 | 51.4832 | 51.6736 | 51.8640 | 52.0544 | 52.2449 | 52.4353 | 52.6257 |
| 52.8162 | 53.0067 | 53.1972 | 53.3877 | 53.5782 | 53.7687 | 53.9593 | 54.1498 | 54.3404 | 54.5310 |
| 54.7216 | 54.9122 | 55.1028 | 55.2934 | 55.4841 | 55.6748 | 55.8654 | 56.0561 | 56.2468 | 56.4375 |
| 56.6282 | 56.8190 | 57.0097 | 57.2005 | 57.3913 | 57.5820 | 57.7728 | 57.9636 | 58.1545 | 58.3453 |
| 58.5362 | 58.7270 | 58.9179 | 59.1088 | 59.2997 | 59.4906 | 59.6815 | 59.8725 | 60.0634 | 60.2544 |
| 60.4454 | 60.6364 | 60.8274 | 61.0184 | 61.2094 | 61.4005 | 61.5916 | 61.7826 | 61.9737 | 62.1648 |
| 62.3559 | 62.5471 | 62.7382 | 62.9293 | 63.1205 | 63.3117 | 63.5029 | 63.6941 | 63.8853 | 64.0765 |
| 64.2678 | 64.4590 | 64.6503 | 64.8416 | 65.0328 | 65.2242 | 65.4155 | 65.6068 | 65.7982 | 65.9895 |
| 66.1809 | 66.3723 | 66.5637 | 66.7551 | 66.9465 | 67.1380 | 67.3294 | 67.5209 | 67.7123 | 67.9039 |
| 68.0954 | 68.2869 | 68.4784 | 68.6700 | 68.8615 | 69.0531 | 69.2447 | 69.4362 | 69.6279 | 69.8195 |
| 70.0111 | 70.2028 | 70.3945 | 70.5861 | 70.7778 | 70.9695 | 71.1612 | 71.3530 | 71.5447 | 71.7365 |
| 71.9282 | 72.1200 | 72.3118 | 72.5036 | 72.6955 | 72.8873 | 73.0792 | 73.2710 | 73.4629 | 73.6548 |
| 73.8467 | 74.0386 | 74.2306 | 74.4225 | 74.6145 | 74.8064 | 74.9984 | 75.1904 | 75.3824 | 75.5745 |
| 75.7665 | 75.9586 | 76.1506 | 76.3427 | 76.5348 | 76.7269 | 76.9190 | 77.1112 | 77.3033 | 77.4955 |
| 77.6877 | 77.8799 | 78.0721 | 78.2643 | 78.4565 | 78.6488 | 78.8410 | 79.0333 | 79.2256 | 79.4179 |
| 79.6102 | 79.8025 | 79.9949 | 80.1872 | 80.3796 | 80.5720 | 80.7643 | 80.9568 | 81.1492 | 81.3416 |
| 81.5341 | 81.7265 | 81.9190 | 82.1115 | 82.3040 | 82.4965 | 82.6891 | 82.8816 | 83.0742 | 83.2667 |
| 83.4593 | 83.6519 | 83.8446 | 84.0372 | 84.2298 | 84.4225 | 84.6152 | 84.8078 | 85.0005 | 85.1933 |
| 85.3860 | 85.5787 | 85.7715 | 85.9642 | 86.1570 | 86.3498 | 86.5426 | 86.7355 | 86.9283 | 87.1212 |
| 87.3140 | 87.5069 | 87.6998 | 87.8927 | 88.0856 | 88.2786 | 88.4715 | 88.6645 | 88.8575 | 89.0505 |
| 89.2435 | 89.4365 | 89.6295 | 89.8226 | 90.0156 | 90.2087 | 90.4018 | 90.5949 | 90.7880 | 90.9812 |
| 91.1743 | 91.3675 | 91.5606 | 91.7538 | 91.9471 | 92.1403 | 92.3335 | 92.5267 | 92.7200 | 92.9133 |

FIG. 3C

EP 0 325 527 A2

4 POTENTIEL $k_m$

| | | | | | | | | | |
|---|---|---|---|---|---|---|---|---|---|
| 93.1966 | 93.2999 | 93.4932 | 93.6865 | 93.8799 | 94.0732 | 94.2666 | 94.4600 | 94.6534 | 94.8468 |
| 95.0403 | 95.2337 | 95.4272 | 95.6206 | 95.8141 | 96.0076 | 96.2012 | 96.3947 | 96.5882 | 96.7818 |
| 96.9754 | 97.1690 | 97.3626 | 97.5562 | 97.7498 | 97.9435 | 98.1371 | 98.3308 | 98.5245 | 98.7182 |
| 98.9119 | 99.1057 | 99.2994 | 99.4932 | 99.6870 | 99.8808 | 100.0746 | 100.2684 | 100.4622 | 100.6561 |
| 100.8500 | 101.0439 | 101.2378 | 101.4317 | 101.6256 | 101.8195 | 102.0135 | 102.2075 | 102.4014 | 102.5954 |
| 102.7894 | 102.9835 | 103.1775 | 103.3716 | 103.5656 | 103.7597 | 103.9538 | 104.1479 | 104.3421 | 104.5362 |
| 104.7304 | 104.9246 | 105.1188 | 105.3130 | 105.5072 | 105.7014 | 105.8957 | 106.0899 | 106.2842 | 106.4785 |
| 106.6728 | 106.8671 | 107.0615 | 107.2558 | 107.4502 | 107.6446 | 107.8390 | 108.0334 | 108.2278 | 108.4223 |
| 108.6167 | 108.8112 | 109.0057 | 109.2002 | 109.3947 | 109.5892 | 109.7838 | 109.9784 | 110.1729 | 110.3675 |
| 110.5621 | 110.7568 | 110.9514 | 111.1461 | 111.3407 | 111.5354 | 111.7301 | 111.9248 | 112.1195 | 112.3143 |
| 112.5090 | 112.7038 | 112.8986 | 113.0934 | 113.2882 | 113.4831 | 113.6779 | 113.8728 | 114.0677 | 114.2626 |
| 114.4575 | 114.6524 | 114.8474 | 115.0423 | 115.2373 | 115.4323 | 115.6273 | 115.8223 | 116.0173 | 116.2124 |
| 116.4075 | 116.6025 | 116.7976 | 116.9927 | 117.1879 | 117.3830 | 117.5782 | 117.7733 | 117.9685 | 118.1637 |
| 118.3590 | 118.5542 | 118.7494 | 118.9447 | 119.1400 | 119.3353 | 119.5306 | 119.7259 | 119.9213 | 120.1166 |
| 120.3120 | 120.5074 | 120.7028 | 120.8982 | 121.0937 | 121.2891 | 121.4846 | 121.6801 | 121.8756 | 122.0711 |
| 122.2666 | 122.4622 | 122.6577 | 122.8533 | 123.0489 | 123.2445 | 123.4401 | 123.6358 | 123.8314 | 124.0271 |
| 124.2228 | 124.4185 | 124.6142 | 124.8100 | 125.0057 | 125.2015 | 125.3973 | 125.5931 | 125.7889 | 125.9847 |
| 126.1806 | 126.3764 | 126.5723 | 126.7682 | 126.9641 | 127.1601 | 127.3560 | 127.5519 | 127.7479 | 127.9439 |
| 128.1399 | 128.3360 | 128.5320 | 128.7280 | 128.9241 | 129.1202 | 129.3163 | 129.5124 | 129.7086 | 129.9047 |
| 130.1009 | 130.2971 | 130.4933 | 130.6895 | 130.8857 | 131.0820 | 131.2783 | 131.4745 | 131.6709 | 131.8672 |
| 132.0635 | 132.2598 | 132.4562 | 132.6526 | 132.8490 | 133.0454 | 133.2418 | 133.4382 | 133.6347 | 133.8312 |
| 134.0277 | 134.2242 | 134.4207 | 134.6173 | 134.8138 | 135.0104 | 135.2070 | 135.4036 | 135.6003 | 135.7969 |
| 135.9935 | 136.1902 | 136.3869 | 136.5836 | 136.7804 | 136.9771 | 137.1739 | 137.3707 | 137.5674 | 137.7643 |
| 137.9611 | 138.1579 | 138.3548 | 138.5517 | 138.7485 | 138.9455 | 139.1424 | 139.3393 | 139.5363 | 139.7333 |
| 139.9303 | 140.1273 | 140.3243 | 140.5213 | 140.7184 | 140.9155 | 141.1126 | 141.3097 | 141.5068 | 141.7040 |
| 141.9011 | 142.0983 | 142.2955 | 142.4927 | 142.6899 | 142.8872 | 143.0845 | 143.2817 | 143.4790 | 143.6764 |
| 143.8737 | 144.0711 | 144.2684 | 144.4658 | 144.6632 | 144.8606 | 145.0581 | 145.2555 | 145.4530 | 145.6505 |
| 145.8480 | 146.0455 | 146.2431 | 146.4406 | 146.6382 | 146.8358 | 147.0334 | 147.2310 | 147.4287 | 147.6263 |
| 147.8240 | 148.0217 | 148.2194 | 148.4171 | 148.6149 | 148.8127 | 149.0104 | 149.2083 | 149.4061 | 149.6039 |
| 149.8017 | 149.9996 | 150.1975 | 150.3954 | 150.5934 | 150.7913 | 150.9892 | 151.1872 | 151.3852 | 151.5832 |
| 151.7813 | 151.9793 | 152.1774 | 152.3755 | 152.5736 | 152.7717 | 152.9698 | 153.1680 | 153.3662 | 153.5643 |
| 153.7626 | 153.9608 | 154.1590 | 154.3573 | 154.5556 | 154.7539 | 154.9522 | 155.1505 | 155.3489 | 155.5473 |
| 155.7457 | 155.9441 | 156.1425 | 156.3409 | 156.5394 | 156.7379 | 156.9364 | 157.1349 | 157.3334 | 157.5320 |
| 157.7305 | 157.9291 | 158.1277 | 158.3264 | 158.5250 | 158.7237 | 158.9224 | 159.1210 | 159.3198 | 159.5185 |
| 159.7173 | 159.9160 | 160.1148 | 160.3136 | 160.5125 | 160.7113 | 160.9102 | 161.1091 | 161.3080 | 161.5069 |
| 161.7059 | 161.9048 | 162.1038 | 162.3028 | 162.5018 | 162.7008 | 162.8999 | 163.0990 | 163.2980 | 163.4971 |
| 163.6963 | 163.8954 | 164.0946 | 164.2938 | 164.4930 | 164.6922 | 164.8915 | 165.0907 | 165.2900 | 165.4893 |
| 165.6886 | 165.8880 | 166.0873 | 166.2867 | 166.4861 | 166.6855 | 166.8849 | 167.0844 | 167.2838 | 167.4833 |
| 167.6829 | 167.8824 | 168.0819 | 168.2815 | 168.4811 | 168.6807 | 168.8803 | 169.0800 | 169.2796 | 169.4793 |
| 169.6790 | 169.8788 | 170.0785 | 170.2783 | 170.4780 | 170.6779 | 170.8777 | 171.0775 | 171.2774 | 171.4773 |
| 171.6772 | 171.8771 | 172.0770 | 172.2770 | 172.4770 | 172.6770 | 172.8770 | 173.0770 | 173.2771 | 173.4772 |
| 173.6773 | 173.8774 | 174.0775 | 174.2777 | 174.4779 | 174.6781 | 174.8783 | 175.0785 | 175.2788 | 175.4791 |
| 175.6794 | 175.8797 | 176.0801 | 176.2804 | 176.4808 | 176.6812 | 176.8816 | 177.0821 | 177.2825 | 177.4830 |
| 177.6835 | 177.8841 | 178.0846 | 178.2852 | 178.4858 | 178.6864 | 178.8870 | 179.0877 | 179.2883 | 179.4890 |
| 179.6897 | 179.8905 | 180.0912 | 180.2920 | 180.4928 | 180.6936 | 180.8945 | 181.0954 | 181.2962 | 181.4971 |
| 181.6981 | 181.8990 | 182.1000 | 182.3010 | 182.5020 | 182.7030 | 182.9041 | 183.1051 | 183.3062 | 183.5074 |
| 183.7085 | 183.9097 | 184.1109 | 184.3121 | 184.5133 | 184.7145 | 184.9158 | 185.1171 | 185.3184 | 185.5198 |
| 185.7211 | 185.9225 | 186.1239 | 186.3253 | 186.5268 | 186.7282 | 186.9297 | 187.1313 | 187.3328 | 187.5343 |
| 187.7359 | 187.9375 | 188.1392 | 188.3408 | 188.5425 | 188.7442 | 188.9459 | 189.1476 | 189.3494 | 189.5512 |
| 189.7530 | 189.9548 | 190.1567 | 190.3586 | 190.5605 | 190.7624 | 190.9643 | 191.1663 | 191.3683 | 191.5703 |
| 191.7724 | | | | | | | | | |

FIG. 3D

1 COURANT

```
-355.0659 -354.7434 -354.4209 -354.0984 -353.7758 -353.4531 -353.1305 -352.8078 -352.4850 -352.1623
-351.8394 -351.5166 -351.1937 -350.8708 -350.5478 -350.2248 -349.9018 -349.5787 -349.2556 -348.9325
-348.6093 -348.2861 -347.9629 -347.6396 -347.3162 -346.9929 -346.6695 -346.3460 -346.0226 -345.6991
-345.3755 -345.0520 -344.7284 -344.4047 -344.0810 -343.7573 -343.4335 -343.1097 -342.7859 -342.4620
-342.1381 -341.8141 -341.4901 -341.1661 -340.8421 -340.5180 -340.1939 -339.8697 -339.5455 -339.2213
-338.8970 -338.5727 -338.2483 -337.9239 -337.5995 -337.2750 -336.9505 -336.6260 -336.3014 -335.9768
-335.6521 -335.3275 -335.0027 -334.6779 -334.3532 -334.0283 -333.7034 -333.3785 -333.0536 -332.7286
-332.4036 -332.0785 -331.7534 -331.4283 -331.1031 -330.7779 -330.4526 -330.1273 -329.8020 -329.4767
-329.1513 -328.8258 -328.5004 -328.1749 -327.8493 -327.5237 -327.1981 -326.8725 -326.5468 -326.2210
-325.8953 -325.5694 -325.2436 -324.9177 -324.5918 -324.2658 -323.9398 -323.6138 -323.2877 -322.9616
-322.6354 -322.3093 -321.9830 -321.6568 -321.3304 -321.0041 -320.6777 -320.3513 -320.0248 -319.6984
-319.3719 -319.0453 -318.7186 -318.3920 -318.0653 -317.7386 -317.4119 -313.8150 -313.4878 -313.1606
-316.1045 -315.7775 -315.4505 -315.1235 -314.7964 -314.4693 -314.1422 -313.8150 -313.4878 -313.1606
-312.8333 -312.5059 -312.1786 -311.8511 -311.5237 -311.1962 -310.8687 -310.5411 -310.2135 -309.8859
-309.5582 -309.2305 -308.9027 -308.5750 -308.2471 -307.9192 -307.5913 -307.2634 -306.9354 -306.6074
-306.2793 -305.9512 -305.6230 -305.2949 -304.9666 -304.6384 -304.3101 -303.9818 -303.6534 -303.3250
-302.9966 -302.6681 -302.3395 -302.0110 -301.6823 -301.3537 -301.0250 -300.6963 -300.3675 -300.0387
-299.7099 -299.3810 -299.0521 -298.7231 -298.3941 -298.0651 -297.7361 -297.4069 -297.0778 -296.7486
-296.4193 -296.0901 -295.7607 -295.4314 -295.1020 -294.7726 -294.4431 -294.1136 -293.7841 -293.4545
-293.1248 -292.7952 -292.4655 -292.1357 -291.8059 -291.4761 -291.1462 -290.8164 -290.4864 -290.1565
-289.8264 -289.4963 -289.1663 -288.8361 -288.5060 -288.1757 -287.8455 -287.5152 -287.1848 -286.8544
-286.5240 -286.1936 -285.8631 -285.5326 -285.2020 -284.8714 -284.5407 -284.2100 -283.8793 -283.5485
-283.2177 -282.8868 -282.5560 -282.2250 -281.8940 -281.5630 -281.2320 -280.9009 -280.5697 -280.2385
-279.9073 -279.5760 -279.2448 -278.9135 -278.5821 -278.2507 -277.9192 -277.5877 -277.2562 -276.9246
-276.5930 -276.2613 -275.9296 -275.5979 -275.2661 -274.9343 -274.6024 -274.2705 -273.9386 -273.6066
-273.2746 -272.9425 -272.6104 -272.2783 -271.9461 -271.6139 -271.2816 -270.9493 -270.6169 -270.2846
-269.9521 -269.6196 -269.2871 -268.9546 -268.6220 -268.2894 -267.9567 -267.6240 -267.2913 -266.9584
-266.6256 -266.2927 -265.9598 -265.6269 -265.2938 -264.9608 -264.6277 -264.2946 -263.9614 -263.6282
-263.2950 -262.9617 -262.6284 -262.2950 -261.9616 -261.6281 -261.2946 -260.9611 -260.6275 -260.2939
-259.9602 -259.6265 -259.2928 -258.9590 -258.6252 -258.2913 -257.9574 -257.6235 -257.2895 -256.9554
-256.6214 -256.2873 -255.9531 -255.6189 -255.2847 -254.9504 -254.6161 -254.2817 -253.9473 -253.6128
-253.2784 -252.9438 -252.6093 -252.2746 -251.9400 -251.6053 -251.2705 -250.9358 -250.6010 -250.2661
-249.9312 -249.5962 -249.2612 -248.9262 -248.5911 -248.2560 -247.9208 -247.5856 -247.2504 -246.9151
-246.5798 -246.2444 -245.9090 -245.5736 -245.2381 -244.9025 -244.5670 -244.2313 -243.8957 -243.5600
-243.2242 -242.8884 -242.5526 -242.2167 -241.8808 -241.5448 -241.2088 -240.8728 -240.5367 -240.2006
-239.8644 -239.5282 -239.1919 -238.8557 -238.5193 -238.1829 -237.8465 -237.5100 -237.1735 -236.8369
-236.5003 -236.1637 -235.8270 -235.4903 -235.1535 -234.8167 -234.4799 -234.1429 -233.8060 -233.4690
-233.1320 -232.7949 -232.4578 -232.1207 -231.7835 -231.4462 -231.1089 -230.7716 -230.4342 -230.0968
-229.7594 -229.4218 -229.0843 -228.7467 -228.4091 -228.0714 -227.7337 -227.3959 -227.0581 -226.7203
-226.3824 -226.0445 -225.7065 -225.3685 -225.0304 -224.6923 -224.3541 -224.0159 -223.6777 -223.3394
-223.0011 -222.6627 -222.3243 -221.9859 -221.6474 -221.3088 -220.9702 -220.6316 -220.2929 -219.9542
-219.6154 -219.2766 -218.9378 -218.5989 -218.2599 -217.9210 -217.5819 -217.2429 -216.9037 -216.5646
-216.2254 -215.8861 -215.5469 -215.2075 -214.8681 -214.5287 -214.1893 -213.8497 -213.5102 -213.1706
-212.8309 -212.4913 -212.1515 -211.8118 -211.4719 -211.1321 -210.7922 -210.4522 -210.1122 -209.7722
-209.4321 -209.0919 -208.7518 -208.4115 -208.0713 -207.7310 -207.3906 -207.0502 -206.7098 -206.3693
-206.0287 -205.6882 -205.3475 -205.0069 -204.6662 -204.3254 -203.9846 -203.6437 -203.3028 -202.9619
-202.6209 -202.2799 -201.9388 -201.5977 -201.2565 -200.9153 -200.5741 -200.2328 -199.8914 -199.5500
-199.2086 -198.8671 -198.5256 -198.1841 -197.8424 -197.5008 -197.1591 -196.8173 -196.4755 -196.1337
-195.7918 -195.4499 -195.1079 -194.7659 -194.4238 -194.0817 -193.7395 -193.3973 -193.0551 -192.7128
-192.3704 -192.0280 -191.6856 -191.3431 -191.0006 -190.6580 -190.3154 -189.9727 -189.6300 -189.2873
```

FIG. 4A

EP 0 325 527 A2

2 COURANT $h_m$

```
                                                      -186.8867 -186.5436 -186.2004 -185.8572
-188.9445 -188.6016 -188.2588 -187.9158 -187.5728 -187.2298 -183.4534 -183.1098 -182.7662 -182.4225
-185.5139 -185.1707 -184.8273 -184.4839 -184.1404 -183.7970 -180.0155 -179.6714 -179.3273 -178.9832
-182.0788 -181.7350 -181.3912 -181.0473 -180.7034 -180.3595 -176.5729 -176.2284 -175.8838 -175.5392
-178.6390 -178.2948 -177.9505 -177.6062 -177.2618 -176.9173 -173.1256 -172.7806 -172.4356 -172.0905
-175.1945 -174.8498 -174.5051 -174.1603 -173.8154 -173.4705 -169.6736 -169.3282 -168.9826 -168.6371
-171.7454 -171.4002 -171.0550 -170.7097 -170.3644 -170.0190 -166.2169 -165.8710 -165.5250 -165.1789
-168.2915 -167.9458 -167.6002 -167.2544 -166.9086 -166.5628 -162.7554 -162.4090 -162.0625 -161.7160
-164.8329 -164.4867 -164.1405 -163.7943 -163.4481 -163.1018 -159.2891 -158.9422 -158.5953 -158.2483
-161.3695 -161.0229 -160.6762 -160.3295 -159.9828 -159.6360 -155.8180 -155.4706 -155.1232 -154.7758
-157.9013 -157.5542 -157.2070 -156.8599 -156.5126 -156.1653 -152.3421 -151.9942 -151.6463 -151.2983
-154.4282 -154.0807 -153.7330 -153.3854 -153.0377 -152.6899 -148.8613 -148.5129 -148.1645 -147.8161
-150.9503 -150.6023 -150.2542 -149.9060 -149.5578 -149.2096 -145.3756 -145.0267 -144.6778 -144.3289
-147.4676 -147.1190 -146.7704 -146.4218 -146.0731 -145.7243 -141.8849 -141.5356 -141.1862 -140.8368
-143.9799 -143.6309 -143.2818 -142.9326 -142.5835 -142.2342 -138.3893 -138.0395 -137.6896 -137.3397
-140.4873 -140.1378 -139.7882 -139.4385 -139.0889 -138.7391 -134.8888 -134.5385 -134.1881 -133.8377
-136.9897 -136.6397 -136.2896 -135.9395 -135.5893 -135.2391 -131.3832 -131.0324 -130.6815 -130.3305
-133.4872 -133.1366 -132.7861 -132.4354 -132.0847 -131.7340 -127.8726 -127.5212 -127.1699 -126.8184
-129.9796 -129.6285 -129.2774 -128.9263 -128.5751 -128.2239 -124.3569 -124.0051 -123.6531 -123.3012
-126.4669 -126.1154 -125.7638 -125.4121 -125.0604 -124.7087 -120.8361 -120.4837 -120.1313 -119.7789
-122.9492 -122.5971 -122.2450 -121.8929 -121.5407 -121.1884 -117.3102 -116.9573 -116.6044 -116.2514
-119.4264 -119.0738 -118.7212 -118.3685 -118.0158 -117.6630 -113.7791 -113.4257 -113.0723 -112.7188
-115.8984 -115.5453 -115.1922 -114.8390 -114.4857 -114.1325 -110.2428 -109.8889 -109.5350 -109.1809
-112.3652 -112.0116 -111.6580 -111.3043 -110.9505 -110.5967 -106.7013 -106.3469 -105.9924 -105.6378
-108.8269 -108.4727 -108.1186 -107.7643 -107.4100 -103.8643 -103.5095 -103.1546 -102.7996 -102.4446 -102.0895
-105.2833 -104.9286 -104.5739 -104.2192 -103.8643 -99.9580 -99.6025 -99.2470 -98.8915 -98.5359
-101.7344 -101.3792 -101.0240 -100.6687 -100.3134 -96.0452 -95.6891 -95.3330 -94.9769
 -98.1802  -97.8245  -97.4687  -97.1129  -96.7571 -96.4011 -92.4824 -92.1259 -91.7693 -91.4126
 -94.6207  -94.2645  -93.9082  -93.5518  -93.1954 -92.8390 -88.9143 -88.5572 -88.2001 -87.8428
 -91.0559  -90.6991  -90.3422  -89.9853  -89.6284 -89.2714 -85.3408 -84.9832 -84.6255 -84.2677
 -87.4856  -87.1282  -86.7709  -86.4134  -86.0559 -85.6984 -81.7618 -81.4036 -81.0453 -80.6870
 -83.9099  -83.5520  -83.1941  -82.8361  -82.4780 -82.1200 -78.1773 -77.8186 -77.4598 -77.1009
 -80.3287  -79.9703  -79.6118  -79.2533  -78.8947 -78.5360 -74.5873 -74.2280 -73.8687 -73.5092
 -76.7420  -76.3830  -76.0240  -75.6649  -75.3058 -74.9466 -70.9917 -70.6319 -70.2720 -69.9120
 -73.1498  -72.7903  -72.4306  -72.0710  -71.7113 -71.3516 -67.3906 -67.0302 -66.6697 -66.3091
 -69.5520  -69.1919  -68.8317  -68.4715  -68.1113 -67.7509 -63.7838 -63.4228 -63.0618 -62.7006
 -65.9485  -65.5879  -65.2272  -64.8664  -64.5056 -64.1447 -60.1713  -59.8098 -59.4181 -59.0645
 -62.3395  -61.9783  -61.6170  -61.2557  -60.8943 -60.5328 -56.5531 -56.1910 -55.8288 -55.4666
 -58.7247  -58.3629  -58.0011  -57.6392  -57.2772 -56.9152 -52.9292 -52.5665 -52.2037 -51.8409
 -55.1043  -54.7419  -54.3795  -54.0170  -53.6544 -53.2919 -49.2995 -48.9362 -48.5728 -48.2094
 -51.4780  -51.1151  -50.7521  -50.3890  -50.0259 -49.6627 -45.6639 -45.3001 -44.9361 -44.5721
 -47.8460  -47.4824  -47.1189  -46.7552  -46.3915 -46.0278 -42.0225 -41.6581 -41.2935 -40.9289
 -44.2081  -43.8440  -43.4798  -43.1156  -42.7513 -42.3869 -38.3752 -38.0102 -37.6451 -37.2799
 -40.5643  -40.1996  -39.8349  -39.4700  -39.1051 -38.7402 -34.7220 -34.3563 -33.9906 -33.6248
 -36.9146  -36.5493  -36.1840  -35.8186  -35.4531 -35.0875 -31.0627 -30.6964 -30.3301 -29.9638
 -33.2590  -32.8931  -32.5271  -32.1611  -31.7950 -31.4289 -27.3974 -27.0306 -26.6636 -26.2967
 -29.5973  -29.2308  -28.8643  -28.4976  -28.1310 -27.7642 -23.7261 -23.3586 -22.9911 -22.6235
 -25.9296  -25.5625  -25.1953  -24.8281  -24.4608 -24.0935 -20.0486 -19.6805 -19.3124 -18.9442
 -22.2558  -21.8881  -21.5203  -21.1525  -20.7846 -20.4166 -16.3650 -15.9963 -15.6275 -15.2586
 -18.5759  -18.2075  -17.8392  -17.4707  -17.1022 -16.7336 -12.6751 -12.3058 -11.9364 -11.5669
 -14.8898  -14.5208  -14.1518  -13.7827  -13.4136 -13.0444
```

FIG. 4B

EP 0 325 527 A2

3 COURANT   $h_m$

| | | | | | | | | | |
|---|---|---|---|---|---|---|---|---|---|
| -11.1974 | -10.8279 | -10.4582 | -10.0885 | -9.7187 | -9.3489 | -8.9790 | -8.6091 | -8.2390 | -7.8690 |
| -7.4988 | -7.1286 | -6.7583 | -6.3880 | -6.0176 | -5.6472 | -5.2766 | -4.9060 | -4.5354 | -4.1647 |
| -3.7939 | -3.4230 | -3.0521 | -2.6812 | -2.3101 | -1.9391 | -1.5679 | -1.1967 | -.8254 | -.4540 |
| -.0826 | .2889 | .6604 | 1.0320 | 1.4037 | 1.7754 | 2.1472 | 2.5191 | 2.8910 | 3.2630 |
| 3.6351 | 4.0072 | 4.3794 | 4.7516 | 5.1240 | 5.4964 | 5.8688 | 6.2413 | 6.6139 | 6.9865 |
| 7.3592 | 7.7320 | 8.1049 | 8.4777 | 8.8507 | 9.2238 | 9.5969 | 9.9700 | 10.3433 | 10.7166 |
| 11.0899 | 11.4633 | 11.8368 | 12.2104 | 12.5840 | 12.9577 | 13.3314 | 13.7053 | 14.0792 | 14.4531 |
| 14.8271 | 15.2012 | 15.5754 | 15.9496 | 16.3239 | 16.6982 | 17.0726 | 17.4471 | 17.8217 | 18.1963 |
| 18.5710 | 18.9457 | 19.3205 | 19.6954 | 20.0703 | 20.4454 | 20.8205 | 21.1956 | 21.5708 | 21.9461 |
| 22.3214 | 22.6969 | 23.0724 | 23.4479 | 23.8235 | 24.1992 | 24.5750 | 24.9508 | 25.3267 | 25.7026 |
| 26.0787 | 26.4547 | 26.8309 | 27.2071 | 27.5834 | 27.9598 | 28.3362 | 28.7127 | 29.0893 | 29.4659 |
| 29.8426 | 30.2194 | 30.5962 | 30.9731 | 31.3501 | 31.7272 | 32.1043 | 32.4814 | 32.8587 | 33.2360 |
| 33.6134 | 33.9909 | 34.3684 | 34.7460 | 35.1236 | 35.5014 | 35.8792 | 36.2570 | 36.6350 | 37.0130 |
| 37.3911 | 37.7692 | 38.1474 | 38.5257 | 38.9041 | 39.2825 | 39.6610 | 40.0396 | 40.4182 | 40.7969 |
| 41.1757 | 41.5545 | 41.9334 | 42.3124 | 42.6915 | 43.0706 | 43.4498 | 43.8290 | 44.2084 | 44.5878 |
| 44.9672 | 45.3468 | 45.7264 | 46.1061 | 46.4858 | 46.8657 | 47.2456 | 47.6256 | 48.0056 | 48.3857 |
| 48.7659 | 49.1461 | 49.5265 | 49.9069 | 50.2873 | 50.6679 | 51.0485 | 51.4291 | 51.8099 | 52.1907 |
| 52.5716 | 52.9526 | 53.3336 | 53.7147 | 54.0959 | 54.4772 | 54.8585 | 55.2399 | 55.6214 | 56.0029 |
| 56.3845 | 56.7662 | 57.1480 | 57.5298 | 57.9117 | 58.2937 | 58.6757 | 59.0579 | 59.4401 | 59.8223 |
| 60.2047 | 60.5871 | 60.9696 | 61.3521 | 61.7348 | 62.1175 | 62.5002 | 62.8831 | 63.2660 | 63.6490 |
| 64.0321 | 64.4152 | 64.7985 | 65.1818 | 65.5651 | 65.9486 | 66.3321 | 66.7157 | 67.0993 | 67.4831 |
| 67.8669 | 68.2508 | 68.6347 | 69.0188 | 69.4029 | 69.7870 | 70.1713 | 70.5556 | 70.9400 | 71.3245 |
| 71.7091 | 72.0937 | 72.4784 | 72.8632 | 73.2480 | 73.6330 | 74.0180 | 74.4031 | 74.7882 | 75.1734 |
| 75.5588 | 75.9441 | 76.3296 | 76.7151 | 77.1007 | 77.4864 | 77.8722 | 78.2580 | 78.6439 | 79.0299 |
| 79.4160 | 79.8021 | 80.1883 | 80.5746 | 80.9610 | 81.3475 | 81.7340 | 82.1206 | 82.5073 | 82.8940 |
| 83.2808 | 83.6677 | 84.0547 | 84.4418 | 84.8289 | 85.2161 | 85.6034 | 85.9908 | 86.3783 | 86.7658 |
| 87.1534 | 87.5411 | 87.9288 | 88.3167 | 88.7046 | 89.0926 | 89.4806 | 89.8688 | 90.2570 | 90.6453 |
| 91.0337 | 91.4221 | 91.8107 | 92.1993 | 92.5880 | 92.9768 | 93.3656 | 93.7546 | 94.1436 | 94.5326 |
| 94.9218 | 95.3111 | 95.7004 | 96.0898 | 96.4793 | 96.8689 | 97.2585 | 97.6482 | 98.0380 | 98.4279 |
| 98.8179 | 99.2079 | 99.5980 | 99.9882 | 100.3785 | 100.7689 | 101.1593 | 101.5498 | 101.9404 | 102.3311 |
| 102.7219 | 103.1127 | 103.5036 | 103.8946 | 104.2857 | 104.6769 | 105.0681 | 105.4595 | 105.8509 | 106.2424 |
| 106.6339 | 107.0256 | 107.4173 | 107.8092 | 108.2010 | 108.5930 | 108.9851 | 109.3772 | 109.7695 | 110.1618 |
| 110.5542 | 110.9466 | 111.3392 | 111.7318 | 112.1245 | 112.5173 | 112.9102 | 113.3032 | 113.6963 | 114.0894 |
| 114.4826 | 114.8759 | 115.2693 | 115.6627 | 116.0563 | 116.4499 | 116.8436 | 117.2374 | 117.6313 | 118.0253 |
| 118.4193 | 118.8135 | 119.2077 | 119.6020 | 119.9963 | 120.3908 | 120.7854 | 121.1800 | 121.5747 | 121.9695 |
| 122.3644 | 122.7594 | 123.1545 | 123.5496 | 123.9448 | 124.3402 | 124.7356 | 125.1310 | 125.5266 | 125.9223 |
| 126.3180 | 126.7139 | 127.1097 | 127.5058 | 127.9019 | 128.2980 | 128.6943 | 129.0906 | 129.4870 | 129.8836 |
| 130.2802 | 130.6768 | 131.0736 | 131.4705 | 131.8674 | 132.2645 | 132.6616 | 133.0588 | 133.4561 | 133.8535 |
| 134.2509 | 134.6485 | 135.0462 | 135.4439 | 135.8417 | 136.2396 | 136.6376 | 137.0357 | 137.4339 | 137.8321 |
| 138.2305 | 138.6289 | 139.0275 | 139.4261 | 139.8248 | 140.2235 | 140.6224 | 141.0214 | 141.4205 | 141.8196 |
| 142.2188 | 142.6182 | 143.0176 | 143.4171 | 143.8167 | 144.2163 | 144.6161 | 145.0160 | 145.4159 | 145.8160 |
| 146.2161 | 146.6163 | 147.0167 | 147.4171 | 147.8175 | 148.2182 | 148.6188 | 149.0196 | 149.4204 | 149.8214 |
| 150.2224 | 150.6236 | 151.0248 | 151.4261 | 151.8275 | 152.2290 | 152.6306 | 153.0323 | 153.4340 | 153.8359 |
| 154.2379 | 154.6399 | 155.0420 | 155.4443 | 155.8466 | 156.2490 | 156.6515 | 157.0541 | 157.4569 | 157.8596 |
| 158.2625 | 158.6655 | 159.0686 | 159.4718 | 159.8750 | 160.2784 | 160.6818 | 161.0853 | 161.4890 | 161.8927 |
| 162.2965 | 162.7004 | 163.1044 | 163.5085 | 163.9128 | 164.3170 | 164.7214 | 165.1259 | 165.5305 | 165.9352 |
| 166.3400 | 166.7448 | 167.1498 | 167.5548 | 167.9600 | 168.3652 | 168.7706 | 169.1760 | 169.5816 | 169.9872 |
| 170.3929 | 170.7988 | 171.2047 | 171.6107 | 172.0168 | 172.4230 | 172.8294 | 173.2358 | 173.6423 | 174.0489 |
| 174.4556 | 174.8624 | 175.2693 | 175.6763 | 176.0834 | 176.4906 | 176.8979 | 177.3052 | 177.7127 | 178.1203 |
| 178.5280 | 178.9358 | 179.3437 | 179.7517 | 180.1598 | 180.5679 | 180.9762 | 181.3846 | 181.7931 | 182.2017 |

FIG. 4C

| | | | | | | | | | |
|---|---|---|---|---|---|---|---|---|---|
| 182.6104 | 183.0191 | 183.4280 | 183.8370 | 184.2461 | 184.6553 | 185.0646 | 185.4739 | 185.8834 | 186.2930 |
| 186.7027 | 187.1125 | 187.5224 | 187.9324 | 188.3425 | 188.7527 | 189.1630 | 189.5734 | 189.9839 | 190.3945 |
| 190.8052 | 191.2160 | 191.6270 | 192.0380 | 192.4491 | 192.8603 | 193.2717 | 193.6831 | 194.0946 | 194.5063 |
| 194.9180 | 195.3299 | 195.7418 | 196.1539 | 196.5660 | 196.9783 | 197.3907 | 197.8032 | 198.2158 | 198.6284 |
| 199.0412 | 199.4541 | 199.8672 | 200.2802 | 200.6935 | 201.1068 | 201.5202 | 201.9338 | 202.3474 | 202.7612 |
| 203.1750 | 203.5890 | 204.0031 | 204.4172 | 204.8315 | 205.2459 | 205.6604 | 206.0750 | 206.4897 | 206.9045 |
| 207.3195 | 207.7345 | 208.1497 | 208.5649 | 208.9803 | 209.3958 | 209.8114 | 210.2271 | 210.6429 | 211.0588 |
| 211.4748 | 211.8909 | 212.3072 | 212.7236 | 213.1400 | 213.5566 | 213.9733 | 214.3901 | 214.8070 | 215.2240 |
| 215.6411 | 216.0584 | 216.4757 | 216.8932 | 217.3108 | 217.7285 | 218.1463 | 218.5642 | 218.9822 | 219.4004 |
| 219.8186 | 220.2370 | 220.6555 | 221.0741 | 221.4928 | 221.9116 | 222.3305 | 222.7496 | 223.1687 | 223.5880 |
| 224.0074 | 224.4269 | 224.8465 | 225.2663 | 225.6861 | 226.1061 | 226.5262 | 226.9464 | 227.3667 | 227.7871 |
| 228.2077 | 228.6284 | 229.0491 | 229.4700 | 229.8910 | 230.3122 | 230.7334 | 231.1548 | 231.5763 | 231.9979 |
| 232.4196 | 232.8415 | 233.2634 | 233.6855 | 234.1077 | 234.5300 | 234.9524 | 235.3750 | 235.7976 | 236.2205 |
| 236.6434 | 237.0664 | 237.4895 | 237.9128 | 238.3362 | 238.7597 | 239.1834 | 239.6071 | 240.0310 | 240.4550 |
| 240.8791 | 241.3034 | 241.7277 | 242.1522 | 242.5769 | 243.0016 | 243.4264 | 243.8514 | 244.2765 | 244.7018 |
| 245.1271 | 245.5526 | 245.9782 | 246.4039 | 246.8298 | 247.2557 | 247.6818 | 248.1081 | 248.5344 | 248.9609 |
| 249.3875 | 249.8142 | 250.2411 | 250.6681 | 251.0952 | 251.5224 | 251.9498 | 252.3773 | 252.8049 | 253.2326 |
| 253.6605 | 254.0885 | 254.5166 | 254.9449 | 255.3733 | 255.8018 | 256.2305 | 256.6592 | 257.0881 | 257.5172 |
| 257.9464 | 258.3756 | 258.8051 | 259.2346 | 259.6643 | 260.0941 | 260.5241 | 260.9542 | 261.3844 | 261.8148 |
| 262.2452 | 262.6758 | 263.1066 | 263.5375 | 263.9685 | 264.3997 | 264.8309 | 265.2623 | 265.6939 | 266.1256 |
| 266.5574 | 266.9893 | 267.4214 | 267.8537 | 268.2860 | 268.7185 | 269.1512 | 269.5840 | 270.0169 | 270.4500 |
| 270.8831 | 271.3164 | 271.7499 | 272.1835 | 272.6172 | 273.0511 | 273.4851 | 273.9193 | 274.3536 | 274.7881 |
| 275.2226 | 275.6573 | 276.0922 | 276.5272 | 276.9623 | 277.3976 | 277.8330 | 278.2686 | 278.7043 | 279.1402 |
| 279.5761 | 280.0123 | 280.4486 | 280.8850 | 281.3216 | 281.7583 | 282.1952 | 282.6321 | 283.0693 | 283.5066 |
| 283.9440 | 284.3816 | 284.8194 | 285.2572 | 285.6953 | 286.1335 | 286.5717 | 287.0102 | 287.4489 | 287.8876 |
| 288.3265 | 288.7656 | 289.2048 | 289.6442 | 290.0837 | 290.5234 | 290.9631 | 291.4031 | 291.8432 | 292.2835 |
| 292.7240 | 293.1645 | 293.6053 | 294.0461 | 294.4872 | 294.9284 | 295.3697 | 295.8112 | 296.2529 | 296.6946 |
| 297.1366 | 297.5787 | 298.0210 | 298.4634 | 298.9060 | 299.3488 | 299.7917 | 300.2347 | 300.6780 | 301.1213 |
| 301.5649 | 302.0086 | 302.4524 | 302.8965 | 303.3406 | 303.7850 | 304.2294 | 304.6741 | 305.1190 | 305.5639 |
| 306.0091 | 306.4543 | 306.8998 | 307.3455 | 307.7913 | 308.2372 | 308.6834 | 309.1297 | 309.5761 | 310.0228 |
| 310.4696 | 310.9166 | 311.3637 | 311.8110 | 312.2585 | 312.7061 | 313.1539 | 313.6019 | 314.0500 | 314.4983 |
| 314.9469 | 315.3955 | 315.8443 | 316.2933 | 316.7425 | 317.1919 | 317.6414 | 318.0911 | 318.5410 | 318.9910 |
| 319.4413 | 319.8917 | 320.3422 | 320.7930 | 321.2439 | 321.6950 | 322.1463 | 322.5978 | 323.0495 | 323.5013 |
| 323.9533 | 324.4055 | 324.8578 | 325.3104 | 325.7632 | 326.2161 | 326.6692 | 327.1225 | 327.5760 | 328.0296 |
| 328.4835 | 328.9375 | 329.3917 | 329.8461 | 330.3008 | 330.7555 | 331.2104 | 331.6657 | 332.1210 | 332.5765 |
| 333.0323 | 333.4882 | 333.9443 | 334.4007 | 334.8572 | 335.3138 | 335.7708 | 336.2279 | 336.6852 | 337.1426 |
| 337.6003 | 338.0582 | 338.5163 | 338.9746 | 339.4331 | 339.8917 | 340.3506 | 340.8097 | 341.2690 | 341.7285 |
| 342.1882 | 342.6481 | 343.1082 | 343.5685 | 344.0291 | 344.4898 | 344.9507 | 345.4119 | 345.8732 | 346.3348 |
| 346.7966 | 347.2586 | 347.7208 | 348.1832 | 348.6458 | 349.1087 | 349.5717 | 350.0350 | 350.4985 | 350.9622 |
| 351.4262 | 351.8903 | 352.3547 | 352.8193 | 353.2841 | 353.7492 | 354.2145 | 354.6800 | 355.1457 | 355.6117 |
| 356.0779 | 356.5443 | 357.0109 | 357.4778 | 357.9449 | 358.4122 | 358.8798 | 359.3477 | 359.8157 | 360.2840 |
| 360.7525 | 361.2213 | 361.6903 | 362.1595 | 362.6290 | 363.0988 | 363.5687 | 364.0390 | 364.5094 | 364.9802 |
| 365.4512 | 365.9224 | 366.3939 | 366.8656 | 367.3376 | 367.8098 | 368.2824 | 368.7551 | 369.2281 | 369.7014 |
| 370.1750 | 370.6488 | 371.1229 | 371.5973 | 372.0719 | 372.5468 | 373.0220 | 373.4974 | 373.9731 | 374.4491 |
| 374.9254 | 375.4019 | 375.8788 | 376.3559 | 376.8333 | 377.3110 | 377.7890 | 378.2673 | 378.7458 | 379.2248 |
| 379.7039 | 380.1834 | 380.6631 | 381.1432 | 381.6236 | 382.1043 | 382.5853 | 383.0666 | 383.5482 | 384.0302 |
| 384.5124 | 384.9950 | 385.4779 | 385.9612 | 386.4447 | 386.9287 | 387.4129 | 387.8975 | 388.3825 | 388.8677 |
| 389.3533 | 389.8393 | 390.3257 | 390.8124 | 391.2995 | 391.7869 | 392.2747 | 392.7630 | 393.2516 | 393.7405 |
| 394.2299 | 394.7197 | 395.2099 | 395.7005 | 396.1915 | 396.6830 | 397.1749 | 397.6673 | 398.1601 | 398.6534 |
| 399.1471 | 399.6414 | 400.1361 | 400.6313 | 401.1270 | 401.6234 | 402.1202 | 402.6176 | 403.1155 | 403.6141 |
| 404.1133 | | | | | | | | | |

FIG. 4D